(19) **Europäisches Patentamt**
**European Patent Office**
**Office européen des brevets**

(11) **EP 2 031 528 A1**

(12) **EUROPEAN PATENT APPLICATION**
published in accordance with Art. 153(4) EPC

(43) Date of publication:
**04.03.2009 Bulletin 2009/10**

(51) Int Cl.:
**G06F 19/00** (2006.01)    **C12N 15/00** (2006.01)

(21) Application number: **07744170.7**

(22) Date of filing: **25.05.2007**

(86) International application number:
**PCT/JP2007/060736**

(87) International publication number:
**WO 2007/139037 (06.12.2007 Gazette 2007/49)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **26.05.2006 JP 2006147433**

(71) Applicant: **Kyoto University**
**Kyoto-shi, Kyoto 606-8501 (JP)**

(72) Inventors:
• **OKUNO, Yasushi**
**Kyoto-shi, Kyoto 606-8501 (JP)**
• **TANEISHI, Kei**
**Kyoto-shi, Kyoto 606-8501 (JP)**
• **TSUJIMOTO, Gozoh**
**Kyoto-shi, Kyoto 606-8501 (JP)**

(74) Representative: **Gill, Stephen Charles et al**
**Mewburn Ellis LLP**
**33 Gutter Lane**
**London**
**EC2V 8AS (GB)**

(54) **ESTIMATION OF PROTEIN-COMPOUND INTERACTION AND RATIONAL DESIGN OF COMPOUND LIBRARY BASED ON CHEMICAL GENOMIC INFORMATION**

(57) This invention is intended to perform the screening of the compound for efficiency to win reasonably. It is solved by the data processing method that the first space expressing coordinates of the first chemical substance group and the second space expressing coordinates of the second chemical substance group are defined, and the first chemical substance group is **characterized by** the first characteristic amount, and the second chemical substance group is **characterized by** the second characteristic amount, the coordinates in the said first space and the coordinates in the said second space are map-transformed by technique chosen from multivariate analysis technique, mechanic learning method and methods equivalent for those. To be concrete, interaction forms of a protein group and compound group are studied by machine learing as a statictic pattern of protein characteristic amount (biological information such as arrangement or the expression information) and compound charcteristic amound ((chemical compound information such as a structure, the physical property) of the compound, and it is the system which realized a interaction prediction based on it, and the hereby problems mentioned above were solved.

[Figure 1]

Fig.1

EP 2 031 528 A1

**Description**

[Technical field]

The present invention relates to a data processing method for efficiently screening using two types of chemical substance databases and for rational design. In detail, the present invention relates to a data processing method for an estimation of compound-protein interaction using both chemical substance information, such as a chemical property of the compound, and biological information, such as sequence information of genes.

[Background technology]

At present when the human genome is disclosed, a pharmaceutical application utilizing the human genome attracts attention. For drug development based upon the genomic information, it is essential to ascertain an interaction between protein (gene) and a compound; however, massive labor is required to experimentally ascertain these interactions. The market in Japan of the bioinformatics is estimated to be sideways movement compared with the previous year for about 35 billion yen in 2004. Of these, after all the market of the package software is sideways movement for about 5 billion yen. However, the market of the pharmacokinetics and a simulation of the examination for toxicity, protein functional analysis software is enlarged (Nikkei bio yearbook 2005). In addition, with another document, global market 2003 of the bioinformatics is estimated to be predictive 2,870 billion yen in predictive 1,770 billion yen, 2015 in 211,600 million yen a year, 2010 (1= non-patent document Nikkei industry newspaper November 29, 2004). A study for this technical application and manufacture is performed in drug industry and a bioventure flourishingly, and medical supplies, the enlarge of the market to bioindustry are anticipated now.

As a prior art for estimating the interaction between protein and a compound, in general, an estimation system using a conformational model is known (Non-patent Literatures 2 to 5). This system is a method to estimate a stable complex structure with ligand and its strength of binding, and it is referred to as a docking study. However, these technique has the problems such as it being impossible to calculate the conjunction of compound group and the protein group having an infinite variation cyclopedically for a prediction to have bases of the science if they do not get a reliable tertiary structure not consisting of X-ray crystallography, the calculation that they used a tertiary structure for more in order that charge is high, and a calculation moment suffers enormously.

Further, with a conventional method (Non-Patent Literature 6) by the inventor himself, after a compound group and a protein group are statistically processed separately, such as cluster analysis, compound processed data and protein processed data are integrated and they are displayed on a two-dimensional map, and an interaction pair of protein and the compound is presumed, and this technique is successful technology for remarkable improvement of the prediction performance by collecting the statistics at the same time, and handling a protein group and compound group.

Besides, in Nature magazine Chemical Space special feature of December, 2004 (non-patent document 7), it is shown which "must intend for only useful compound group for a creature because it is impossible the variation of the compound is enormous, and to pursue all compounds", but the report about the method of the concreteness is not done so far. Because this technique build a statistical model of the interaction form of a protein group and the compound group, it succeeds in statistics modeling of the chemistry character of the useful compound group for a creature.

Pinpointed the high active domain of the chemical space, and published Japanese translation of PCT International Publication No. 2002-530,727 publication bulletin (patent document 1) lists what a library builds. However, this method defines chemical space with only chemical information (structure activity correlation information, pharmacophore information). However, by this method, principal component analysis (PCA) is used.

NIH of the US has started a chemical genomic project as a national project in 2004. Since then, the application of the genomic information to a chemical field has been focused upon over the world but mainly in the US and Europe. Therefore, at least in the developed countries, such as the US, an efficient estimation method is in demand.

[Non-patent document 1]Nikkei industry newspaper November 29, 2004

[Non-patent document 2]Yoshifumi Fukunishi, Yoshiaki Mikami, and Haruki Nakamura "Thefillingpotential method:A method for estimating the free energy surfaceforprotein-liganddocking" J.Phys. Chem. B. (2003) 13210. 107, 13201-

[Non-patent document 3]Shoichet, B.K.,D.L. Bodian,and I.D. Kuntz, "Molecular docking usingshapedescriptors." J.Comp. 1992. Chem., 13 (3), 380-397.

[Non-patent document 4]Jones G, WillettP, Glen RC, Leach AR,Taylor R. "Development and validation ofa geneticalgorithm for flexible docking. "J Mol Biol.1997.267(3): 727-748

[Non-patent document 5]Rarey M, KramerB,Lengauer T. "Time-efficient docking of flexibleligands intoactive sites ofproteins." Proc Int Conf IntellSyst Mol Biol.1995; 3:300-308.

[Non-patent document 6]Okuno, Y.,Yang,J., Taneishi, K., Yabuuchi,H., Tsujimoto, G, "GLIDA:GPCR-Liganddatabasefor Chemical Genomic Drug Discovery" Nucleic AcidsResearch,34,D673-677, 2006

[non-patent document 7] "Chemical Space", 432 Nature, No 7019(Insight) 823-865

[Patent document 1] published Japanese translation of PCT International Publication 2002-530,727 publication bulletin

[Disclosure of the invention]

[Problem to be solved by the invention]

This invention is intended to perform the screening of the compound for efficiency to win reasonably.

[Means for solving the problem]

The inventors solved it so that as a result of having developed it zealously, and defined the second space that expressed the first space and coordinates in space of the second chemical substance group to express coordinates in space of the first chemical substance group, and the first chemical substance group was characterized by the first characteristic amount, and the second chemical substance group was characterized by the second characteristic amount, and a correlation with the first above space and the second above space became greatest because representation converted a coordinate of the first said space and a coordinate of the second said space by technique chosen from multivariate analysis technique, mechanic learning method and those equivalence of value methods group. Machine learning did interaction form of a protein group and the compound group as a pattern of statistics of characteristic amount (biological information such as arrangement or the expression information) of the protein and the characteristic amount (chemical compound information such as a structure, the physical property) of the compound, and it was the system which realized a interaction prediction to be based on it, and the hereby problems mentioned above were solved to be concrete.

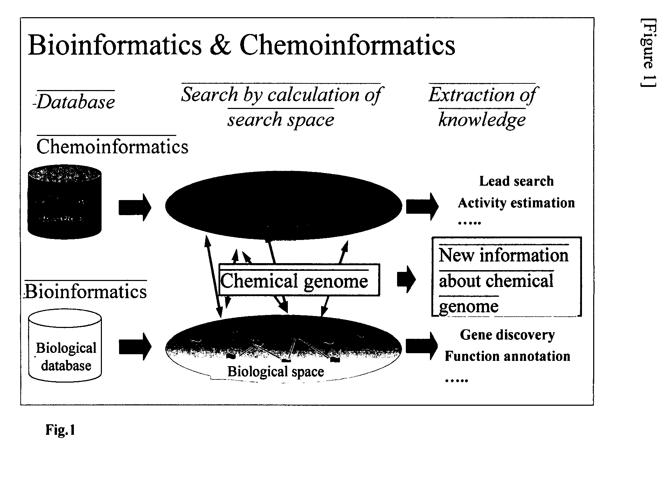

The conventional law in a library design and the lead search of the compound used only the chemical information of the compound like the figure 1 upper section (Chemoinformatics). Let the bioinformatics technology of the figure 1 lower berth fuse, and, as for the technique of these inventors, conventional technique only for this chemical information was able to develop new technique called a compound library design and the lead search that took genome information into account.

Coordinate space showing the relative locus correlation (similarity) of the individual compound is necessary for the way of thinking that is basic when performing a lead search and the library design of the compound by a calculation.

For example, the coin on the chemical space of the figure 1 upper section expresses a different compound each, and, as for the characteristic compound resembling, it is said called chemical space that the coordinate space which consists of the locus of these compounds if posted in the relatively near locus correlation so that it is.

The thing which expressed a similar correlation for relative locus relations about the gene (protein) is bio space likewise; (the figure 1 lower berth, a square seal, gene or protein). They can make the model which fused between these chemical space and bio space because a link (figure 1, a central arrow) does a bond of the individual compound and protein.

Therefore, this invention offers the following.

(1) A data structure that it is data structure possessing the second space defined by the first space defined by the database of the coordinates in space of the first chemical substance and database of the coordinates in space of the second chemical substance, and the first chemical substance is characterized by the first characteristic amount, and the second chemical substance is characterized by the second characteristic amount.

(2) A data structure of the description in item 1 characterized by that correlate simple each other is not seen with the first quantity of character and characteristic amount of the second above mentioned above.

(3) A characteristic amount of and the quantity of first character mentioned above is chemical character of the first chemical substance mentioned above above the second is data structure of the description in item 1 which is the biological activity of the chemical compound of the second above.

(4) A data structure of the description in item 1 where the first chemical substance mentioned above is a compound, and the second chemical substance mentioned above is biological substance.

(5) A data structure of the description above that biological substancee is nucleic acid, peptide or item 4 chosen from polypeptide or protein, saccharide or polysaccharide, lipid and those complexes group.

(6) A coordinates in space of the biological substance mentioned above are data structures of the description in item 4 defined by information of at least one kind chosen from the arrangement information, secondary structure, tertiary structure, quaternary structure, tertiary structure information, expression information, pass way information, function information and biological activity information group.

(7)It is data structure of the description in item 1 where a coordinate of the first space mentioned above and a coordinate of the second above space are defined so that the first space mentioned above and the correlation with the second space mentioned above become maximum.

(8)With the first space mentioned above and the second space mentioned above, it is data structure of the description in item 1 characterized by what is defined by technique chosen from multivariate analysis technique, mechanic learning method and those equivalent methods group.

(9)The multivariate analysis technique mentioned above is data structure of the description in canonical correlational analysis (CCA) and item 8 chosen from the kernel canonical correlational analysis (kernel CCA) group.

(10)The mechanic learning method mentioned above is data structure of the description in item 8 including the support vector machine (SVM) law.

(11)The coordinates in space of the first chemical substance mentioned above are data structures of the description in chemical information and item 1 defined by information chosen from the chemical character group.

(12)It is data structure of the description in item 9 where the chemical information mentioned above is defined by a compound descriptor.

(13)The compound descriptor mentioned above is data structure of the description in one-dimensional descriptor, two-dimensional descriptor and item 12 chosen from the three-dimensional descriptor group.

(14)The compound descriptor mentioned above is data structure of the description in and it is a one-dimensional descriptor item 12 which it is characteristic of that the one-dimensional descriptor mentioned above describes chemical composition.

(15)The compound descriptor mentioned above is data structure of the description in and it is a two-dimensional descriptor item 12 characterized by that the two-dimensional descriptor mentioned above describes chemical topology.

(16)The compound descriptor mentioned above is data structure of the description in and it is a three-dimensional descriptor item 12 characterized by that the three-dimensional descriptor mentioned above describes character chosen from the three-dimensional configuration and sensualism group.

(17)It is data structure of the description in item 12 where the compound descriptor mentioned above is pharmacophore.

(18)The compound descriptor mentioned above is pharmacophore, and the pharmacophore mentioned above includes at least three spatially remote pharmacophore center; each pharmacophore center,

(i)With space locus,

(ii)At least leading a hydrogen bond acceptor, a hydrogen bond donor, a negative charge in an implication, the pharmacophore model of the unit set with an appointed pharmacophore model to specify a certain chemical character, and it is data structure of the description in positive charge center, hydrophobicity center, aromatic series center and item 12 where the default category that does not enter the model of which pharmacophore of others either is included in.

(19)It is data structure of the description in item 18 to give the space locus mentioned above for sequestration distance between adjacent pharmacophore center or a sequestration distance range.

(20)Be a method to produce chemical compounds having desired character,

A) A process to offer the included first chemical substance group to the first space defined by the database of the coordinates in space of the first chemical substance

B) The process when it is in a process to offer the included second chemical substance group to the second space defined by the database of the coordinates in space of the second chemical substance, and the first chemical substance is characterized by the first characteristic amount, and the second chemical substance is characterized by the second characteristic amount

C) The process that chooses desired character in quantity of second character

D) The process that calculates a focused region in the second space of the second chemical substance having the chosen desired character mentioned above

E) The process that calculates a target region of the first space that there is in focused region and less than mentioned above predetermined distance

F) The process that chooses the chemical compound which there is in the target region of the first space mentioned above
A method to include these.

(21)It is a method of the description in item 20 which includes a training process more to let first space and the second space mentioned above relate to with sample data.

(22)When, by the training mentioned above, generated line A and line B, and the correlation between YB which expressed the first space of XA and the second modality to express the first space of the first modality became greatest, and a move of character from the first above modality to the second modality was enabled, and, as for the above move, the entry of the chemical compound hereby expressed the heterogeneous data (for example, compound and protein) of two kinds that chemical compound information lined up in a line with line X,Y (the first space, line X, the second space, line Y) in line X, a line of Y,

[Number 1]

$$X = n \times p \qquad Y = n \times q \qquad n \geq p \geq q \geq 1$$

$$f = Xa \qquad g = Yb$$

$$\sigma_f{}^2 = \frac{1}{n-1} f'f \qquad \sigma_{fg} = \frac{1}{n-1} f'g \qquad \sigma_g{}^2 = \frac{1}{n-1} g'g$$

in order to maximize the correlation between the first space and the second space, a set of coefficient vectors a and b to maximize the correlation is searched, and
[Number 2]

$$r_{fg} = \frac{\sigma_{fg}}{\sigma_f \sigma_g}$$

herein,
[0018]

[Number 3]

$$\sigma_f^2 = \sigma_g^2 = 1$$

with the following conditions:,
[0020]

[Number 4]

$$r_{fg} = \sigma_{fg}$$

is maximized,
[0022]

[Number 5]

$$r_{fg}$$

is referred to as canonical correlation, and [0024]

[Number 6]

$$f, g$$

A method to call a plus associate variable.

(23)In the plus associate correlation analysis mentioned above, to perform peculiar value resolution of X and Y,

[Number 7]

$$X = U_X D_X V'_X \quad Y = U_Y D_Y V'_Y$$

$$U'_X U_Y = UDV'$$

U, D and V are calculated, and using U, D and V, and the following are acquired:
[Number 8]

$$A = \sqrt{n-1} V_X D_X^{-1} U \quad B = \sqrt{n-1} V_Y D_Y^{-1} V$$

$$F = \sqrt{n-1} U_X U \quad G = \sqrt{n-1} U_Y V$$

it is provided that A, B, F and G are as follows:
[Number 9]

$$f_i = Xa_i \qquad g_i = Yb_i \qquad i = 1, \ldots, q$$

$$A = \lfloor a_1, \ldots, a_q \rfloor \qquad B = \lfloor b_1, \ldots, b_q \rfloor$$

$$F = \lfloor f_1, \ldots, f_q \rfloor \qquad G = \lfloor g_1, \ldots, g_q \rfloor$$

$$F = XA \qquad G = YB$$

herein, the higher correlation can be obtained from i=1 in order [0029]

[Number 10]

$$r_{f\,g}$$

It is a method of the description in item 22 characterized by a thing.

(24)When a chisel is given the query of XA expressing the first space mentioned above as a result of above query of YB expressing the second space mentioned above; is a method of the description in item 19 characterized by that can identify it because YB has XA and the greatest correlation.

(25)It is a method of the description in item 20 including A) - F) by a mechanic learning method automatically in the process mentioned above.

(26)It is a method of the description in item 25 where the mechanic learning method mentioned above is achieved by SVM method.

(27)In the mechanic learning method mentioned above,

G1) The process that lets machine learning algorithm train by symphysis data with known first chemical compound and the second chemical substance;

G2) The process that I refer, and maps a pair with respect in space model built by the database of the coordinates in space of the first chemical substance and the database of the coordinates in space of the second chemical substance with first chemical compound and the second chemical substance becoming the inquiry ; or

G3) When there is the inquiry pair mentioned above in space area, judge first chemical compound and the second chemical substance to be connected, and there is not it in space area; the process that the first chemical substance and the second chemical substance judge not to be connected

It is a method of the description in item 25.

(28)It is a method of the description in item 20 characterized by not related to the first quantity of character and characteristic amount of the second above mentioned above each other.

(29)The characteristic amount of and the quantity of first character mentioned above is chemical character of the first chemical substance mentioned above above the second is a method of the description in item 20 which is the biological activity of the chemical compound of the second above.

(30)The chemical compound of and the first chemical substance mentioned above is a compound above the second is a method of the description in item 20 where it is biological substance.

(31)The biological substance mentioned above is a method of the description in nucleic acid, peptide or item 30 chosen from polypeptide or protein, saccharide or polysaccharide, lipid and those complexes group.

(32)The coordinates in space of the biological substance mentioned above are methods of the description in item 30 defined by information of at least one kind chosen from the arrangement information, secondary structure, tertiary structure, quaternary structure, tertiary structure information, expression information, pass way information, function information and biological activity information group.

(33)The characteristic amount of and the quantity of first character mentioned above is creature character of the first chemical substance mentioned above above the second is a method of the description in item 20 which is the chemistry activity of the chemical compound of the second above.

(34)The second chemical substance mentioned above is a method of the description in and it is a compound item 20 where the first chemical substance mentioned above is biological substance.

(35)The biological substance mentioned above is a method of the description in nucleic acid, peptide or item 34 chosen from polypeptide or protein, saccharide or polysaccharide, lipid and those complexes group.

(36)The coordinates in space of the biological substance mentioned above are methods of the description in item 30 defined by information of at least one kind chosen from the arrangement information, secondary structure, tertiary structure, quaternary structure, tertiary structure information, expression information, pass way information, function information and biological activity information group.

(37)It is a method of the description in item 20 where the first coordinate mentioned above and the second above coordinate are defined so that the first correlation with space and the second space mentioned above becomes maximum.

(38)With the first space mentioned above and the second space mentioned above, it is a method of the description in item 20 characterized by what is defined by technique chosen from multivariate analysis technique, mechanic learning method and those equivalent methods group.

(39)The multivariate analysis technique mentioned above is a method of the description in canonical correlational analysis (CCA) and item 38 chosen from the kernel canonical correlational analysis (kernel CCA) group.

(40)The mechanic learning method mentioned above is a method of the description in item 38 including the support vector machine (SVM) law.

(41)Furthermore, it is a method of the description in item 38 characterized by putting more than two together among multivariate analysis technique, a mechanic learning method or equivalent methods and applying it.

(42)The correlation mentioned above is a method of the description and item 20 which are a correlation between the focused region mentioned above and the target region mentioned above.

(43)Furthermore, it is a method of the description in item 20 including a process producing the chosen chemical compounds mentioned above in Silico

(44)Furthermore, it is a method of the description in item 20 including the process to produce the chosen chemical compounds mentioned above with wet.

(45)It is a method of the description in item 44 which the wet production mentioned above uses combinatorial chemistry, and is achieved.

(46)It is a method of the description in item 44 which the wet production mentioned above uses gene-recombination technology, and is achieved.

(47)Furthermore, it is a method of the description in item 20 which it measures the characteristic amount of the second above of the chemical compound of the first above space after the selection of the chemical compound of the first space mentioned above, and includes a process choosing a chemical compound having really desired activity more.

(48)It is a method of the description in item 20 characterized by accompanied with score based on the second characteristic amount mentioned above and do the chemical compound mentioned above in the selection process of the chemical compound mentioned above.

(49)The coordinates in space of the biological substance mentioned above are methods of the description in item 20 defined by information of at least one kind chosen from the arrangement information, secondary structure, tertiary structure, quaternary structure, tertiary structure information, expression information, pass way information, function information and biological activity information group.

(50)The coordinates in space of the first chemical substance mentioned above are methods of the description in chemical information or item 20 defined by chemical character.

(51)It is a method of the description in item 50 where the chemical information mentioned above is defined by a compound descriptor.

(52)The compound descriptor mentioned above is a method of the description in one-dimensional descriptor, two-dimensional descriptor and item 51 chosen from the three-dimensional descriptor group.

(53)The compound descriptor mentioned above is a method of the description in and it is a one-dimensional descriptor item 51 which it is characteristic of that the one-dimensional descriptor mentioned above describes chemical composition.

(54)The compound descriptor mentioned above is a method of the description in and it is a two-dimensional descriptor item 51 characterized by that the two-dimensional descriptor mentioned above describes chemical topology.

(55)The compound descriptor mentioned above is a method of the description in and it is a three-dimensional descriptor item 51 characterized by that the three-dimensional descriptor mentioned above describes character chosen from the three-dimensional configuration and sensualism group.

(56)It is a method of the description in item 51 where the compound descriptor mentioned above is pharmacophore.

(57)The first chemical substance mentioned above is pharmacophore, and the pharmacophore mentioned above includes at least three spatially remote pharmacophore center; each pharmacophore center,

(i)With space locus,

(ii)At least leading a hydrogen bond acceptor, a hydrogen bond donor, a negative charge in an implication, the pharmacophore model of the unit set with an appointed pharmacophore model to specify a certain chemical character, and it is a method of the description in positive charge center, hydrophobicity center, aromatic series center and item 20 where the default category that does not enter the model of which pharmacophore of others either is included in.

(58)It is a method of the description in item 57 to give the space locus mentioned above for sequestration distance between adjacent pharmacophore center or a sequestration distance range.

(59)The chemical compound which was produced in either Clause 1 of 20-58 items by a method of the description.

(60) A method to produce compound libraries,

A) A process to offer the included first chemical substance group to the first space defined by the database of the coordinates in space of the first chemical substance

B) The process when it is in a process to offer the included second chemical substance group to the second space defined by the database of the coordinates in space of the second chemical substance, and the first chemical substance is characterized by the first characteristic amount, and the second chemical substance is characterized by the second characteristic amount

C) The process of choosing desired character in quantity of second character

D) The process of calculating a focused region in the second space of the second chemical substance having the chosen desired character mentioned above

E) The process of calculating a target region of the first space that there is in focused region and less than mentioned above predetermined distance

F) The process of producing the libraries by choosing the plural chemical compounds which there is in the target region of the first space mentioned above, and have desired character

A method to include these

(61)It is a method of the description in item 60 having character of the description in either Clause 1 of 21-59 items.

(62)The library produced by a method in the descriptionof item 60 or 61

(63) A program to let a computer carry out a method to produce chemical compounds having desired character; and the above method :

A) A process of offering the included first chemical substance group to the first space defined by the database of the coordinates in space of the first chemical substance
B) The process when it is in a process to offer the included second chemical substance group to the second space defined by the database of the coordinates in space of the second chemical substance, and the first chemical substance is characterized by the first characteristic amount, and the second chemical substance is characterized by the second characteristic amount
C) A process of choosing desired character in quantity of second character
D) A process of calculating a focused region in the second space of the second chemical substance having the chosen desired character mentioned above
E) A process of calculating a target region of the first space that there is in focused region and less than mentioned above predetermined distance
F) A process of choosing the chemical compound which there is in the target region of the first space mentioned above A program to include these.

(64) A the computer reading possible recording medium which stored a program to let a computer carry out a method to produce chemical compounds having desired character; and the above method :

A) A process to offer the included first chemical substance group to the first space defined by the database of the coordinates in space of the first chemical substance

B) A process when it is in a process to offer the included second chemical substance group to the second space defined by the database of the coordinates in space of the second chemical substance, and the first chemical substance is characterized by the first characteristic amount, and the second chemical substance is characterized by the second characteristic amount

C) A process of choosing desired character in quantity of second character

D) A process of calculating a focused region in the second space of the second chemical substance having the chosen desired character mentioned above

E) A process of calculating a target region of the first space that there is in focused region and less than mentioned above predetermined distance

F) A process of choosing the chemical compound which there is in the target region of the first space mentioned above A recording medium to include these.

(65) A recording medium that it was the data structure which possessed the second space defined by the first space defined by the database of the coordinates in space of the first chemical substance and database of the coordinates in space of the second chemical substance, and the first chemical substance was characterized by the first characteristic amount, and data structure characterized by the second characteristic amount was recorded as for the second chemical substance.

(66) A system producing chemical compounds having desired character; and the system mentioned above :

A) The first chemical substance group included in the first space defined by the database of the coordinates in space of the first chemical substance

B) The second chemical substance group that it is the second chemical substance group included in the second space defined by the database of the coordinates in space of the second chemical substance, and the first chemical substance is characterized by the first characteristic amount, and the second chemical substance is characterized by the second characteristic amount

C) The tool which chooses desired character in quantity of second character

D) The tool which calculates a focused region in the second space of the second chemical substance having the chosen desired character mentioned above

E) The tool which calculates a target region of the first space that there is in focused region and less than mentioned above predetermined distance

F) The tool which chooses the chemical compound which there is in the target region of the first space mentioned above

G) The tool which produces the chosen chemical compounds mentioned above A system comprising these

(67) A system screening a chemical compound having desired character; and the system mentioned above :

A) The first chemical substance group included in the first space defined by the database of the coordinates in space of the first chemical substance
B) The second chemical substance group that it is the second chemical substance group included in the second space defined by the database of the coordinates in space of the second chemical substance, and the first chemical substance is characterized by the first characteristic amount, and the second chemical substance is characterized by the second characteristic amount
C) The tool which chooses desired character in quantity of second character
D) The tool which calculates a focused region in the second space of the second chemical substance having the chosen desired character mentioned above
E) The tool which calculates a target region of the first space that there is in focused region and less than mentioned above predetermined distance
F) The tool which chooses the chemical compound which there is in the target region of the first space mentioned above A system comprising these

(68) A method to design a chemical compound library,

A) A process of specifying the first space defined by the database of the coordinates in space of the first chemical substance,

B) A process when it is in the process when at least one character identifies the second space defined by the database of the coordinates in space of the known second chemical compound, and the first chemical substance is characterized by the first characteristic amount here, and the second chemical substance is characterized by the second characteristic amount,

C) A process that defines a coordinate of the first space mentioned above again so that the first space mentioned above and the correlation with the second space mentioned above become maximum,

D) A process generating the first space that I defined again mentioned above as a new chemical compound library,

A method to include these

(69) A data processing method to define a interaction pattern between the first chemical substance group and the second chemical substance group as a statistical model, the first space expressing coordinates of the first chemical substance group and the second space expressing coordinates of the second chemical substance group are defined, and the first chemical substance group is characterized by the first characteristic amount, and the second chemical substance group is characterized by the second characteristic amount,

(I) the first chemical substance group is a compound, and the second chemical substance group is a nucleic acid or protein or those complexes, and the first characteristic amount is expressed as a vector consisting of the one kind chemical information of the said first chemical substance group, and the second characteristic amount is expressed as a vector consisting of the one kind biological information of the said second chemical substance group; or

(II) the first chemical substance group is a nucleic acid or protein or those complexes, and the second chemical substance group is a compound, and the first characteristic amount is expressed as a vector consisting of the information of one kind of biological information of the said fist chemical substance group, and the second characteristic amount is expressed as a vector consisting of the one kind chemical information of the said second chemical substance group;

the said first space and the said second space are map-transformed by technique chosen from multivariate analysis technique, mechanic learning method and methods equivalent for those,

the coordinates in the said first space and the coordinates in the said second space are map-transformed by technique chosen from multivariate analysis technique, mechanic learning method and methods equivalent for those, and the map-transformed first space coordinates and the map-transformed second space coordinates are difined.

(70) A data processing method to predict interaction between the first chemical substance and the second chemical substance in a data processing method of the description to (69), including,

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to (69);

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) A process of outputting the first chemical substance and the second chemical substance having more than the specific scores.

(71) A data processing method to produce a chemical substance having a desied characteristic amount or to design library in a data processing method of the description to (69), including,

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to (69);

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

(72) A data processing method to define a interaction pattern between the first chemical substance group and the second chemical substance group as a statistical model, comprising,
the pair of the first chemical substance and the second chemical substance is expressed as the vector that is a combination of the first characteristic amount of the first chemical substance with the second characteristic amount of the second chemical substance,

(I) the first chemical substance group is a compound, and the second chemical substance group is a nucleic acid or protein or those complexes, and the first characteristic amount is expressed as a vector consisting of the one kind chemical information of the said first chemical substance group, and the second characteristic amount is expressed as a vector consisting of the one kind biological information of the said second chemical substance group; or

(II) the first chemical substance group is a nucleic acid or protein or those complexes, and the second chemical substance group is a compound, and the first characteristic amount is expressed as a vector consisting of the information of one kind of biological information of the said fist chemical substance group, and the second characteristic amount is expressed as a vector consisting of the one kind chemical information of the said second chemical substance group;

the vector that is a combination of the first characteristic amount of the first chemical substance with the second characteristic amount of the second chemical substance, is map-transformed into a feature space by technique chosen from multivariate analysis technique, mechanic learning method and methods equivalent for those.

(73) A data processing method to predict interaction between the first chemical substance and the second chemical substance in a data processing method of the description to (72), including,

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to (72);

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction.

D) a process of outputting the first chemical substance and the second chemical substance having more than the specific scores.

and the information of interaction is defined by the information chosen from existence / nothing of binding, biding activity, the pharmacology activity.

(74) A data processing method to produce a chemical substance having a desied characteristic amount or to design library in a data processing method of the description to (72), including,

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to (72);

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction,

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

(75) A data processing device to predict interaction between the first chemical substance and the second chemical substance, using a data processing method of the description to (69), has an arithmetic device and make the said arithmetic device run the method including A)-D);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to (69);

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) A process of outputting the first chemical substance and the second chemical substance having more than the specific scores.

(76) A data processing device to produce a chemical substance having a desied characteristic amount or to design library, using a data processing method of the description to (69), has an arithmetic device and make the said arithmetic device run the method including A)-G);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to (69);

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

(77) A data processing device to predict interaction between the first chemical substance and the second chemical substance, using a data processing method of the description to (72), has an arithmetic device and make the said arithmetic device run the method including A)-D);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to (72);

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction.

D) a process of outputting the first chemical substance and the second chemical substance having more than the specific scores.

and the information of interaction is defined by the information chosen from existence / nothing of binding, biding activity, the pharmacology activity.

(78) A data processing device to produce a chemical substance having a desied characteristic amount or to design library, using a data processing method of the description to (72), has an arithmetic device and make the said arithmetic device run the method including A)-G);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to (72);

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction,

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

(79) A data processing program to predict interaction between the first chemical substance and the second chemical substance, using a data processing method of the description to (69), make calculator run the method including A)-D);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to (69);

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) A process of outputting the first chemical substance and the second chemical substance having more than the specific scores.

(80) A data processing program to predict interaction between the first chemical substance and the second chemical substance, using a data processing method of the description to (69), make calculator run the method including A)-G);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to (69);

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

(81) A data processing program to predict interaction between the first chemical substance and the second chemical substance, using a data processing method of the description to (72), make calculator run the method including A)-D);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to (72);

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction.

D) a process of outputting the first chemical substance and the second chemical substance having more than the specific scores.

and the information of interaction is defined by the information chosen from existence / nothing of binding, biding activity, the pharmacology activity.

(82) A data processing program to predict interaction between the first chemical substance and the second chemical substance, using a data processing method of the description to (72), make calculator run the method including A)-G);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to (72);

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction,

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

(83) A recording medium that stored the data processing program according to any one of (79)-(82), that computer can read.

(84) The chemosynthesis device that has the data processing program according to any one of (79)-(82), and synthesize a chemical substance or a chemical substance group which is prediceted or produced by the said data processing program; or that synthesiza a chemical substance or a chemical substance group which is prediceted or produced by the data processing device according to any one of (75)-(78)

(85) A screeing device with the data processing program according to any one of (79)-(82) that has a way to synthesize a chemical substance in the frist space, assay the second cheracteristic amount of the chemical substance in the said first space, and actually choose the chemical substance which have desired activity.

(86) A chemical substance which is produced by the method data processing program according to any one of (79)-(82), a recording medium of (83), or the device of (84) or (85).

[Effect of the invention]

According to the present invention, a calculation technique for estimating the interaction between a compound and protein using both chemical substance information, such as chemical characteristics of compounds, and biological information, such as genomic information, was able to be developed. In this technique, unlike the conventional type estimation using only chemical characteristic information of compounds, the accuracy of the estimation was successfully improved by adding sequence information of genes.
Therefore, this calculation technique is applicable to the following two:

1) A rational design based upon bioactivity of compound library can be realized; and

2) A search for lead compound with better performance than the conventional method can be provided.

Hereafter, preferred embodiments of the present invention are described, and it should be recognized that a person with ordinary skills in the art pertaining to the present invention can appropriately implement the embodiments from the description of the present invention and publicly-known conventional technology in the technical field, and can easily understand the operation and effect of the present invention.
Therefore, it is understood that advantages of the present invention are evident to a person with ordinary skills in the art pertaining to the present invention by reading and understanding the detailed description mentioned below with reference to attached documents as occasion demands.

[Brief desdription of the figures]

[Figure 1]
Figure 1 is a conception diagram of this invention.
The conventional technique in a library design and the lead compound search was used only the chemical information of the compound indicated in the figure 1 upper section (Chemoinformatics). The technique of this invention is integration of this conventional technique using only chemical information into the bioinformatics technology in the lower stage of Fig. 1, and a new technique of compound library design and lead compound search that took genome information into account was developed.

[Figure 2]

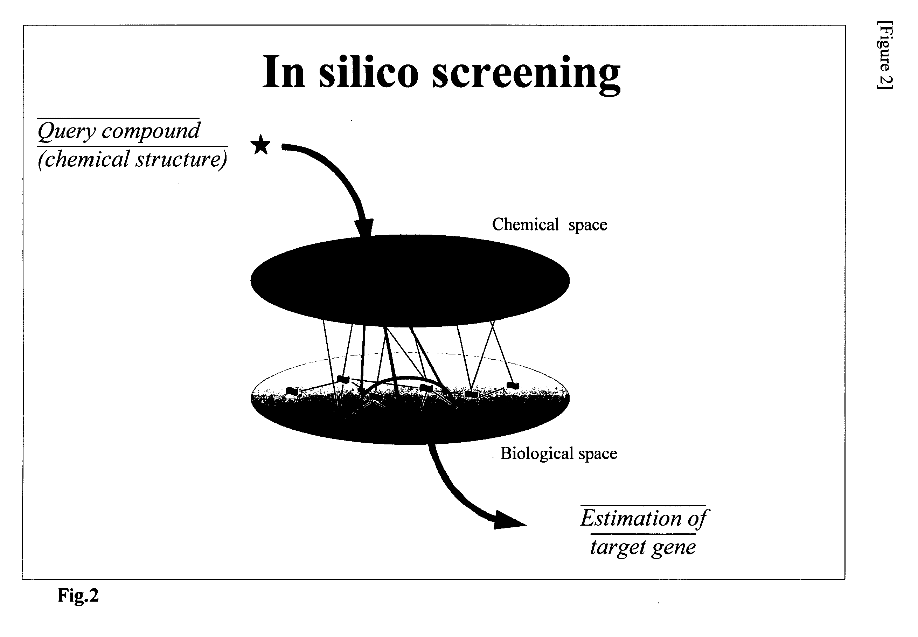

Figure 2 is other conception diagram of this invention. The coin on the chemical space of the upper section expresses a different compound each. The compounds which have similar character is positioned, and the coordinate space which is consistant of positions of these compounds is called chemical space. In the same way, it is bio space to express analogous relationship of gene (protein) as a relative postion (the lower stage, a square mark expresses gene or protein). Moreover the model of integration between chemical space and bio space is able to be made by linking each compound to the protein that the compounds can combine.

[Figure 3]

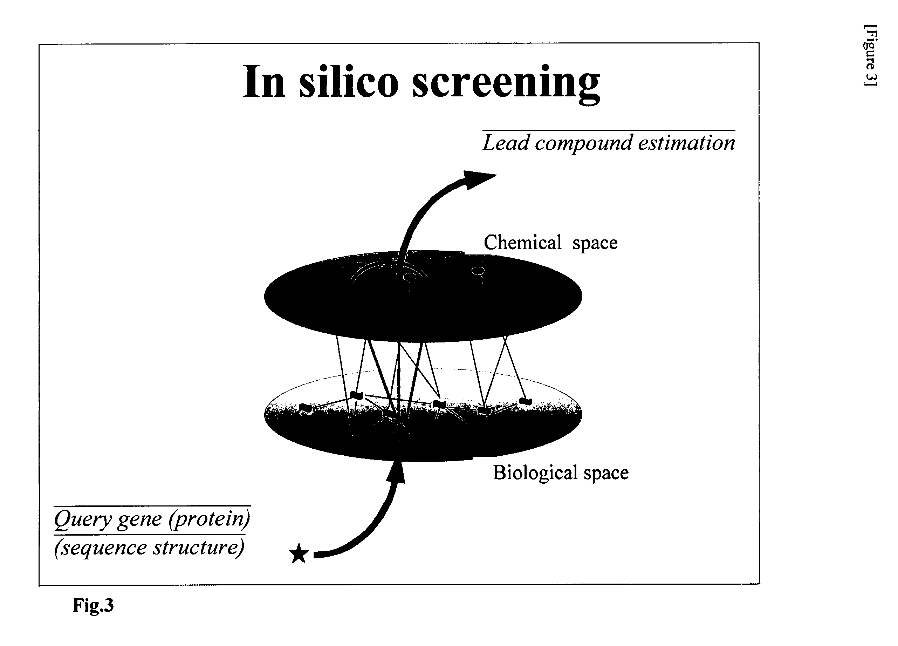

Figure 3 is other conception diagram of this invention. The coin on the chemical space of the upper section expresses a different compound each. The compounds which have similar character is positioned, and the coordinate space which is consistant of positions of these compounds is called chemical space. In the same way, it is bio space to express analogous relationship of gene (protein) as a relative postion (the lower stage, a square mark expresses gene or protein). Moreover the model of integration between chemical space and bio space is able to be made by linking each compound to the protein that the compounds can combine.

[Figure 4]

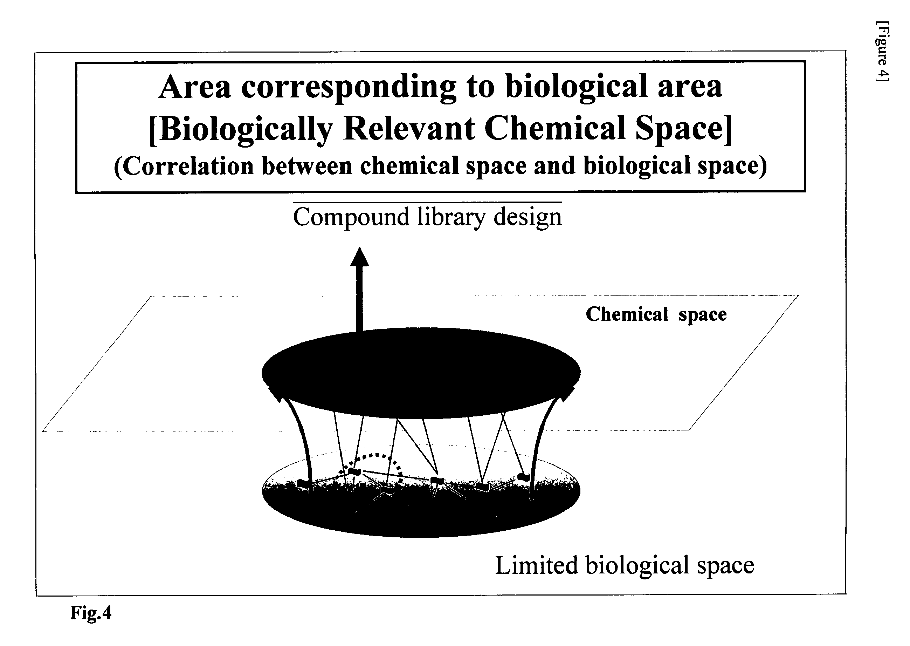

Figure 4 is a conception diagram of this invention. As the figure indicates, compounds in the area which correspond to the yellow bio space (biologically relevant chemical space) are thought to have possibilities of interacting with protein group which consist bio space. Thus, the compound library with biological activity is able to be designed.

[Figure 5]

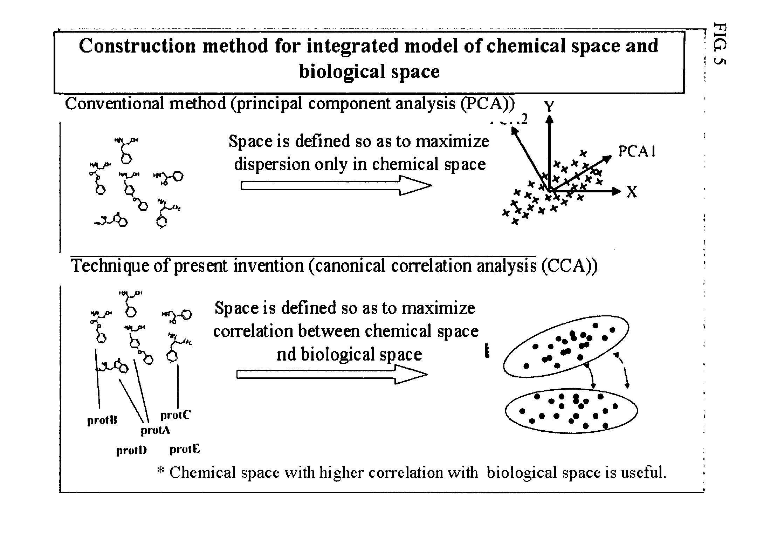

Figure 5 shows character of this technique about the organization method of the most important "integrated model of chemical space and biological space ". The prior technique (the upper section) defined coordinates of chemical space, using only chemical information so that the chemical characters of the compound become various as possible. Here, the big problem is that there is no causal association between the variation of compounds and biological activities. Therefore this technique uses both the chemical information and protein arrangement information, and defines each other's coordinates in space so that a correlation of both space of chemical space and the protein space rose. If the chemical space coordinates are difined with consideration of relevance with biological space, space coordinates which is more convenient for biological activity can be built.

[Figure 6]

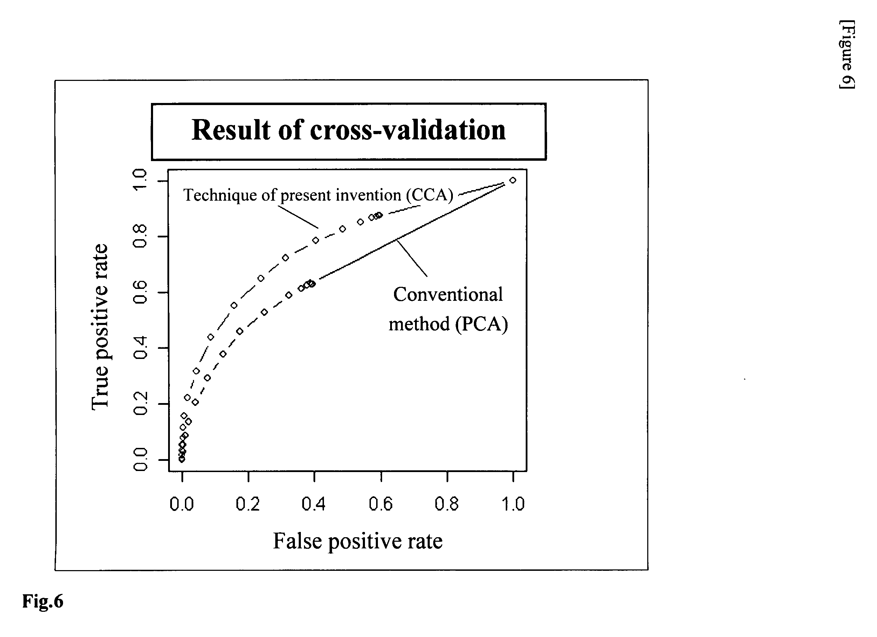

Figure 6 shows the result of in-silico screening conducted using "integrated model of chemical space and biological space", in order to evaluate the performance of the conventional method (PCA) and the present technique (CCA), constructed by both methods, respectively. The figure shows a ROC curve, which is one of the methods to evaluate the estimation performance. This graph indicates that the more upper the curve is positioned, the better the estimation performance is, and since the curve of the present technique is positioned more upper than that of the conventional method, the estimation performance of the present technique is higher than that of the conventional method.

[Figure 7A]

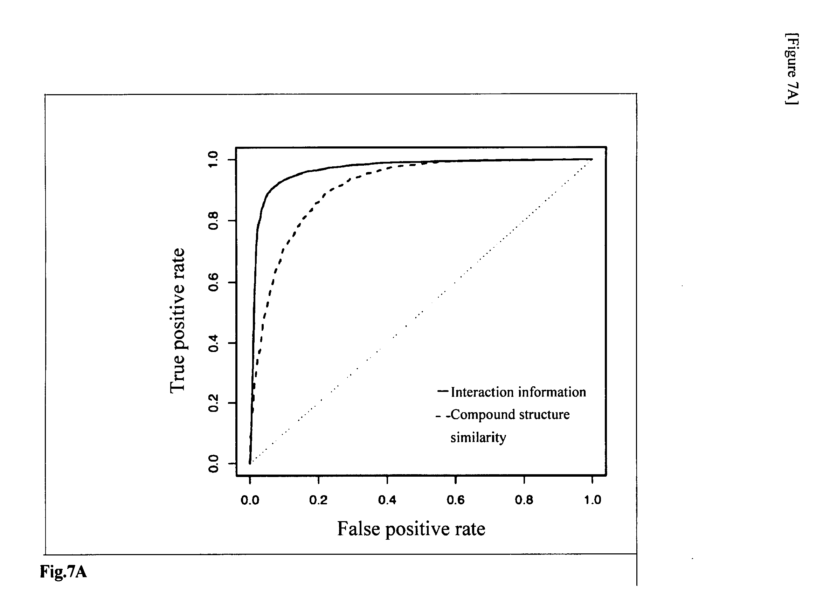

Figure 7A shows an ROC curve for the purpose of the predictive performance comparison between models

[Figure 7B]

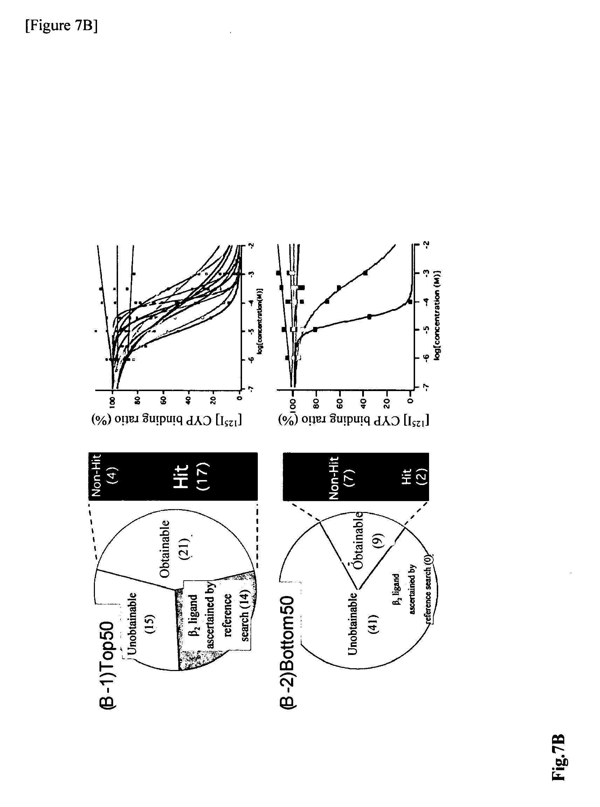

Figure 7B shows results of verification of β2AR ligand estimation results, and also shows results of examination and experiment to compounds in the top 50 (B-1) and bottom 50 (B-2) of estimation score. The left side shows a breakdown of the compounds ascertained in the literature search and the experimental verification, and the right side indicates a binding inhibition curve to [125I]-cyanopindolol, and the vertical axis indicates an inhibition ratio and the horizontal axis indicates a concentration of each compound.

[Figure 7C]

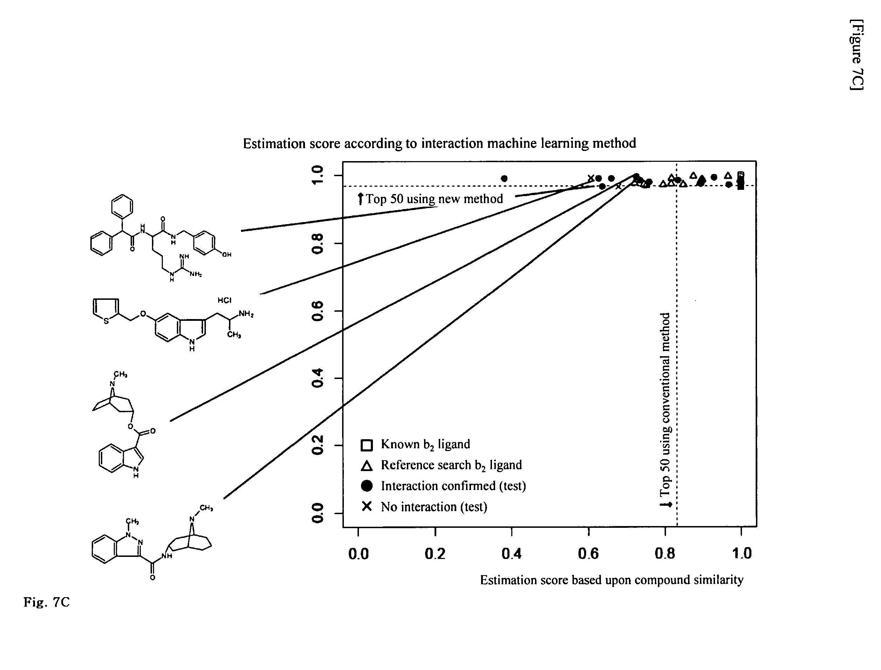

Figure 7C shows a comparison of the β2AR ligand estimation results between new model and a conventional method. Each point is a compound, and the vertical axis indicates a new model and the horizontal axis shows an interaction estimation score by the conventional method. Results of bibliography survey and experiment inspection are displayed by the following classification.

[Figure 8]
It is a conception diagram of this invention. Because the performance of the "in silico screening" prediction was better than prior technique, the designation of compound library (the predication of the target gene) is performed by this technique.
With 6,391,005 compounds in the American NCBI / PubChem database, the protein candidates which interacts with those compounds were predicted and the compound library was designed

[Figure 9]

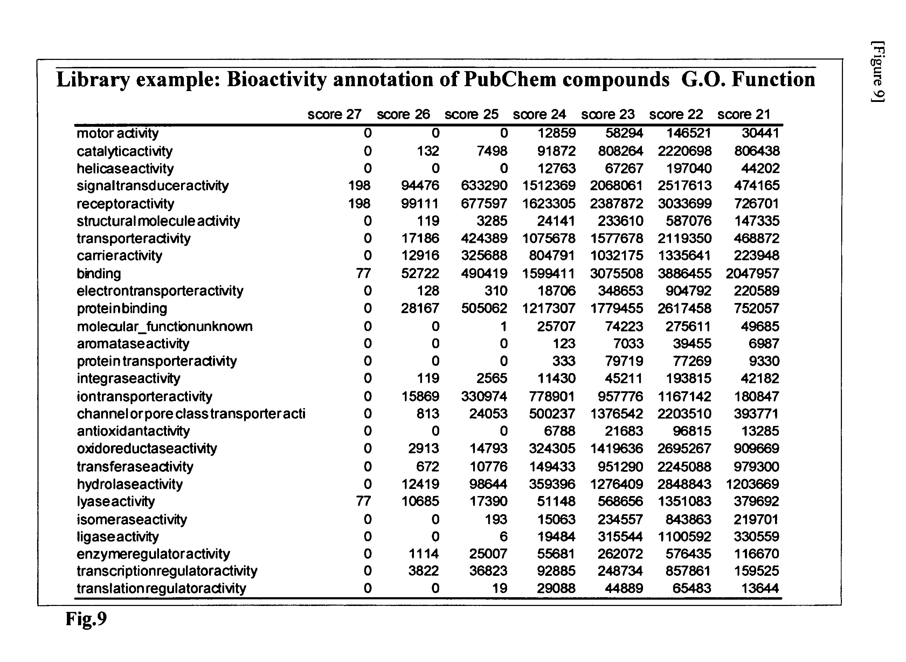

Figure 9 shows results of the bioactivity estimation of PubChem compound. Each row shows data per score indicating the reliability of binding possibility between a compound and a target protein. In other words, it appears that the higher the score becomes, the higher the reliability of the bioactivity of the compound. Further, the items in each line indicate the classification per function of the target protein (based upon gene ontology). The numerical values in the table represent the number of (estimated) compounds corresponding to the correspondence portion. For example, protein regarding the receptor activity is targeted, and for compounds showing score value: 27 or more reliability, 198 compounds are estimated.

[Figure 10]

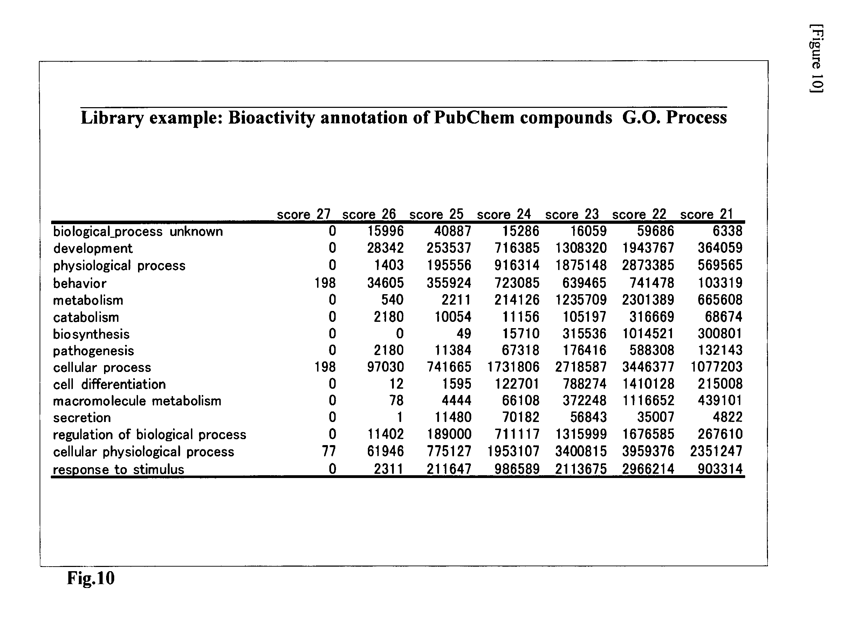

Fig. 10, as similar to Fig. 9, shows results of bioactivity estimation of PubChem compounds, and the function of the target protein is classified using different references, respectively. The view of Fig. 10 is similar to Fig. 9.

[Figure 11]
A calculation flow on the occasion of applying CCA in the present invention.

[Figure 12]
A calculation flow on the occasion of applying SVM in the present invention.

[Figure 13]

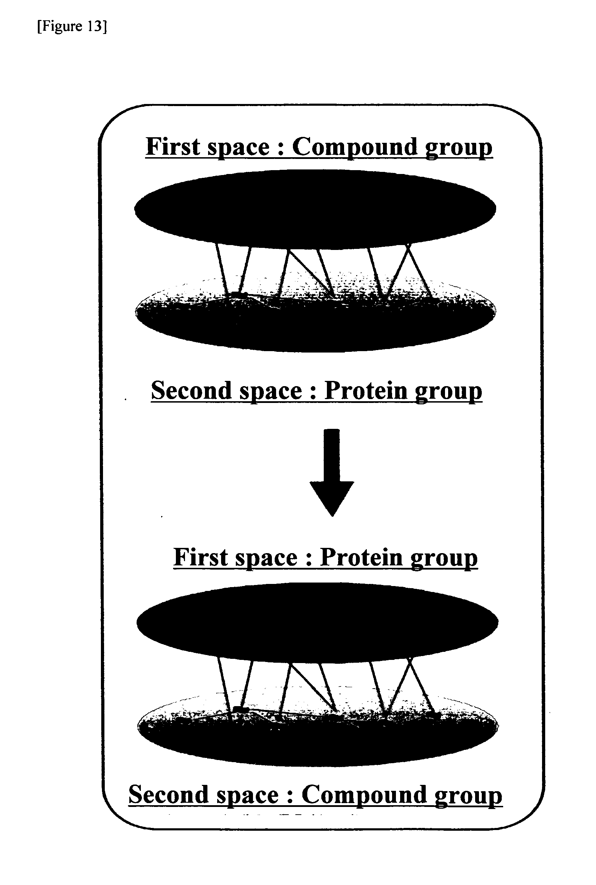

Figure 13 is a conception diagram of this invention.

[The best form to carry out invention]

Hereafter, the present invention will be described. It should be understood throughout the entire specification that the expression as a singular form also includes a concept of its plural form as long as not particularly mentioned. Therefore, articles or adjectives of a singular form (for example, in the case of English, "a", "an" and "the") should be understood as including the concept of its plural form as long as particularly mentioned. Further, terminology used in the specification should be understood as used with a meaning normally used in the technical field as long as particularly mentioned. Therefore, as long as defined in other field, all technical terms and engineering and scientific terms used in the present specification have the same meanings as generally understood by a person with ordinary skills in the art pertaining to the present invention. If the meaning is inconsistent, the one in the present specification (including definition) has priority.

(Definition)

Definitions of the terms that are used below in an instant specification in particular are enumareted.
"Chemical substance" in the present specification is a term in the case of handling a substance especially from an chemical standpoint among general terms of substances, and is referred to as a substance having any certain molecular structure.
"Space coordinates" in the specification are an index for specifying a position of a target within space, "space" is alias of an assembly, and it assumes a type of phase or geometric framework. The space is defined, for example, from chemical substance information or biological information.

"Space" used in this specification is used as replaceable with "Space", and is alias of an assembly, and it assumes a type of phase or geometric framework. The space is defined, for example, from chemical substance information or biological information.As the space, chemical space defined from chemical substance information, such as a compound descriptor or chemical characteristics, expression information, pathway information, functional information and biological space (Current Opinion in Chemical Biology 2005,9: 296-303) defined from biological information, such as bioactivity can be exemplified.

"Characteristic" in the specification means a special feature of a chemical substance. The characteristic, for example, can include physical properties, such as a melting point, a boiling point or specific gravity; chemical properties, such as reactivity, acidity or alkalinity; and biological properties, such as a protein conformation, enzyme activity, binding capacity with a receptor, cytokine capacity, or interaction force between cells, can be exemplified; however, the characteristics are not limited to these.

In the specification, "simple relevancy cannot be confirmed" between the first characteristic and the second characteristic means that no intuitive correlation is confirmed between both. When a vector indicating the first characteristic is regarded as a, b and a vector indicating the second characteristic is x, y, it is assumed that the pairs of a and x, a and y, b and x and b and y have no intuitive correlation. However, there is a case that correlation is confirmed between linear combination vectors m*a+n*b and M*x+N*y (m, n, M and N are coefficients other than 0). In other words, even if a simple relevancy is not confirmed between the first characteristic and the second characteristic, there is a case that a correlation is confirmed between F (a, b, ...) and F' (x, y, ...) where an appropriate conversion is added.

"Biological material" in the specification means an arbitrary substance relating to organisms. The biological material is also regarded as a type of chemical substance. "Biological object" in the specification means a biological organic body, and it includes animals, plants, fungi and virus; however, it is not limited to these. The biological material includes protein, polypeptide, oligopeptide, peptide, polynucleotide, oligonucleotide, nucleotide, nucleic acid (for example, including DNA, such as cDNA or genome DNA, and RNA, such as mRNA), polysaccharide, oligosaccharide, lipid, and complex molecule of these (such as glycolipid, glycoprotein or lipoprotein); however, it is not limited to these. Normally, the biological material can be nucleic acid, protein, lipid, sugar, proteolipid, lipoprotein, glycoprotein and proteoglycan.Preferably the biological substance includes a nucleic acid (DNA or RNA) or protein. In another preferred embodiment, biological substance is a nucleic acid (the DNA which, for example, was composed by genomic DNA or cDNA or PCR). In other preferred embodiments, biological substance can be protein. Preferably such a biological substance can be hormone or cytokine. "The cytokine" used in an instant specification is used in the field concerned; is defined like the meaning of the wide sense most, and it is said with the biologically active substance which is produced by a cell, and acts on the same cell which or is different. Generally it is protein or polypeptide, and the cytokine has control effect of the immune response, the accommodation of the endocrine system, the accommodation of the nervous system, antitumor action, antiviral action, accommodation operation of the cell proliferation, accommodation operation of the cell differentiation. With the instant specification, the cytokine can be protein form or nucleic acid form or other form, but, in points in time to really act, cytokine usually means protein form. "The growth factor" used in this specification is the materials that promote the growth of the cell or call material to control The growth factor is said to be a growth factor or the growth factor. The growth factor is added in a culture medium in cell culture or tissue culture and can replace it by operation of the blood serum macromolecule material. As for many growth factors, it is recognized that it functions as the differentiation regulator that is in a state besides the growth of the cell. Interleukin, chemokine, a hematopoietic factor such as the colony stimulating factor, a tumor necrosis factor, interferon are included in cytokine typically. As a growth factor, a thing having the growth activity that seems to be a platelet-derived growth factor (a PDGF), an epidermal growth factor (EGF), fibroblasts growth factor (FGF), a hepatic mesenchymal cell growth factor (HGF), a vascular endothelial growth factor (VEGF) is raised typically.

"The receptor" in this specification exists in cells or nucleus and has binding capacity for the factor from the outside world or the factor of the intracellular and says the monad which signal is transmitted to by the symphysis. Receptors usually take the form of the protein. Symphysis partners of the receptors usually say ligand.

"The agonist" in this specification binds to a receptor of a certain organism active substance (ligand) with and say the operation that the material has and a factor same (or similar), and to show operation.

"The antagonist" in this specification works antagonistically, and itself says the factor that does not show a physiological function through the receptor to the symphysis to a receptor of a certain organism active substance (ligand). An opiate antagonist, blocker (a blocker), the inhibitor (inhibitor) are included by this antagonist, too.

"Compound" in the specification means a pure substance that can be broken down into single bodies of two types or more of elements due to a chemical change. This may also be referred to as a pure substance that is generated by a chemical binding with atoms in two types or more of elements. Normally, a relative proportion of each element is uniform in general in accordance with the law of definite composition; however, a compound that creates stable crystal even if the relative proportion is continuously changed within a certain range, like a non-stoichiometric compound, is also included in the category of a compound in the specification.I have specific activity to be intended in the set of a certain compound with "the chemical combination foundation" in an instant specification, and it is said about one kind of compound having desired character. For example, as for such single compound, it can be called chemical combination foundation when a compound regulating activity of the biological substance which there is in the aggregation of a compound regulating activity of a certain biological substance is identified. With the instant specification, it is called merely compound.

In the specification, "simple relevancy cannot be confirmed" between the first characteristic and the second characteristic means that no intuitive correlation is confirmed between both. When a vector indicating the first characteristic is regarded as a, b and a vector indicating the second characteristic is x, y, it is assumed that the pairs of a and x, a and y, b and x and b and y have no intuitive correlation. However, there is a case that correlation is confirmed between linear combination vectors m*a+n*b and M*x+N*y (m, n, M and N are coefficients other than 0). In other words, even if a simple relevancy is not confirmed between the first characteristic and the second characteristic, there is a case that a correlation is confirmed between F (a, b, ...) and F' (x, y, ...) where an appropriate conversion is addedFor example, it is not said that simple correlate is not seen because I am related to molecular weight and a melting point or the boiling point to some extent.

To "convert coordinates" "so as to maximize the correlation" in the specification means a situation where the relationship between each element in one space and each element in another space is correlated at maximum as a whole. This definition can be accomplished by various calculation techniques.

"Correlation" in the specification generally means a relevancy between/among two or more variates in mathematic statistics and biostatistics, and means a linear covariant relationship between two probability variates. In the numeric taxonomy, this can be expressed with a similar coefficient (degree of similarity) between two operational taxonomic units (OTU) to be a target. Therefore, the correlation analysis is a statistical strategy to verify whether or not there is correlation based upon a pair of data or to estimate the magnitude of correlation.

"Coefficient of correlation" in the specification means an invariant quantity with a linear transformation indicating the correlation between two probability variables X and Y. This is a value where covariance of X and Y is divided by a square root of product of variance of X and Y, respectively.

For "correlation function", there are space correlation function and time correlation function. An average value A(r)A(r') of a product of physical quantities A(r) and A(r') at two points is referred to as a space correlation function. When this exponentially varies relative to the distance between the two points, this is expressed as follows:

[Number 101]

$$\overline{A(r)A(r')} \propto \exp\left(-\frac{|r-r'|}{\xi}\right)$$

Then, the correlation distance $\xi$ is defined. For the time correlation function, the correlation time $\tau$ is similarly defined. The correction distance $\xi$ and the correction time $\tau$ diverge at a critical point.

Then, the correlation distance $\xi$ is defined. For the time correlation function, the correlation time $\tau$ is similarly defined. The correction distance $\xi$ and the correction time $\tau$ diverge at a critical point.

As a method to maximize the correlation in the specification, a multivariate analysis technique and a mechanical learning method, such as canonical correlation analysis (CCA), kernel canonical correlation analysis (kernel CCA) or support vector machine (SVM), are available.

"Chemical information" in the specification means information as a compound of chemical substance. To be defined in more detail, it includes chemical structure itself and various descriptors calculated by calculation processing from a chemical structure (literature: Handbook of Chemoinformatics: From Data to Knowledge Gasteiger, Johann (EDT) / Publisher: John Wiley Published 2003/10). Normally, information is expressed as a numerical value or a numerical value sequence (vector), respectively. Each compound is expressed as a numerical value sequence (vector) where numerical value sequences of various pieces of appropriately-selected chemical substance information are linked.

Normally, the chemical substance information is defined by a compound descriptor. As the descriptor, for example, one selected from a group comprising a one-dimensional descriptor, a two-dimensional descriptor and a three-dimensional descriptor can be exemplified. "Descriptor" includes a method to describe certain information and the expressed matter. The descriptor can be expressed by radio waves, magnetic waves, sound, lights, colors, images, numerical values, characters or a combination of these. The identification of descriptor group to characterize a molecular structure is an important process in the process to analyze many compounds. Although many descriptors are proposed, these can be classified according to the approach to the molecular structure (see M.Hassan et al., Molecular Diversity, 1996, 2, 64; M.J. McGregor et al., J.Chem. Inf. Comput. Sci., 1999, 39,569; R.D. Brown, Perspectives in Drug Discovery and Design, 1997, 7/8, 31. They are incorporated in the specification as a reference at first. See R.D. Brown et al., J. Chem. Inf. Comput. Sci. 1996, 36, 572; R.D. Brown et al. J. Chem. Inf. Comput. Sci. 1996, 37, 1; D.E. Patterson et al., J. Med. Chem. 1996, 39, 3049; S.K. Kearsley et al., J. Chem. Inf. Comput. Sci. 1996, 36, 118. They are incorporated in the specification as a reference). The one-dimensional (1D) characteristic indicates overall molecular characteristic, such as molecular weight or clogP. The two-dimensional (2D) characteristic includes functionality and connectivity of molecules. As an example of the 2D descriptor, MDL substructure key (see MDL Information System Inc., 14600 Catalina St., San Leandro, CA 94577; M.J. McGregor et al., J. Chem. Inf. Comput. Sci., 1997, 37, 443. They are incorporated in the specification as a reference) and MSI50 descriptor (Molecular Simulations Inc., 9685 Scranton Road, San Diego, CA 92121-3752) are exemplified. For example, useful and publicly-known five rules (rule of five) on the occasion of identifying elements to a drug compound are derived from the one-dimensional descriptor and the two-dimensional descriptor (see C.A. Lipinski et al., Advanced Drug Delivery Reviews, 1997,23,3. This is incorporated in the specification as a reference).

For the calculation of three-dimensional (3D) descriptor, at least one three-dimensional structure having appropriate energy is required. In addition, the three-dimensional descriptor may be calculated by taking contribution from a plurality of conformations into consideration. Further, a descriptor may be selected based upon an important characteristic in the ligand binding or according to other important desired characteristic. Alternatively, in the case of using many descriptors for the analysis of many compound groups, the minimum number of important descriptors should be obtained by a statistical tool, such as principal component analysis (PCA) or partial least squares (PLS).

"Bio information" or "biological information" in the specification means information relating to a biological characteristic, which is a biological material, such as gene or protein, in a chemical compound. The biological information includes, for example, base sequence information of gene, primary structural information, secondary structural information, tertiary structural information, quaternary structure information, conformational information, manifestation information, pathway information, function information and bioactivity information. In the present invention, this biological information is expressed as a numerical value sequence (vector) having numerical values digitalized by calculation processing or measurement as each element.

Various kinds of detection methods and detection tool can be used as far as system or it can detect information caused by a factor to interact by the analysis of this invention. When a creature or a cell is intended for, for example, viewing, a light microscope, a fluorescence microscope, the reading device, with a laser light source for, surface plasmon resonance (SPR) imaging, electric signal, the chemistry or the biochemical marker or a method to use plural number seed for and tool are used as such a detection method and detection tool, but it is not limited to them.

The characteristic of the biological substance can be expressed by a unique descriptor using the biological characteristic as an index. Therefore, in the specification, "index" regarding one state is a function to be a mark for expressing said state. In the specification, for example, in the case of organism or cell, various physical indexes of the organisms or inside the cell (potential, in vivo temperature, traveling speed/distance, localization rate, ellipticity, elongation rate and revolution speed); chemical index (genome dosage, quantity of transcript of specific gene (for example, mRNA, translated protein, modified protein after translation, ion concentration and pH), quantity of metabolic product and ion content); and biological index (such as individual difference, evolutionary rate or drug response); or reactivity or resistance properties, such as environment of the organism or cell, for example, temperature, humidity (for example, absolute humidity or relative humidity), pH, salt concentration (for example, concentration of entire salt or concentration of specific salt), nutrition (for example, quantity of vitamin, quantity of lipid, quantity of protein, quantity of carbohydrate and metallic ion concentration), metal (for example, quantity of entire metal or concentration of specific metal (for example, heavy metal or light metal)), gas (for example, quantity of entire gas or quantity of specific gas (for example, oxygen, carbon dioxide or hydrogen)), organic solvent (for example, quantity of entire organic solvent or concentration of specific organic solvent (for example, ethanol, DMSO or methanol)), pressure (for example, local pressure or overall pressure (atmospheric pressure or water pressure)), viscosity, flow rate (for example, flow rate of a culture medium in the case that the organism exist in the culture medium, membrane flow), luminosity (a quantity of one specific wavelength), light wavelength (for example, other than a visible light, ultraviolet rays and infrared rays can be included), electromagnetic wave, radiation, gravity, tension, sound waves, organisms other than the target organism (for example, parasite, disease-causing bacterium, bacteria, virus), chemical drugs, (for example, drugs, food additives, agricultural chemical, fertilizer, environmental endocrine disrupter, antibiotics, natural products, psychological stress and physical stress can be used as "index" regarding the state.

"Indication", "a display" and "the presentation" in this specification are used exchangealbe and they say that a signal is converted to express so that a sense organ (for example, visual sense, audition, olfaction) can feel it. Rep-

resently they mean, for example, expressing visually, and display indicates measure of displaying the signal visually, when they are used in restricted meanings especially. Thus, "indication", "a display" and "the presentation" are to embody the discriptors or information from them, which are gained by the method of this invention, into the information processed form directly or indirectly. These form for display is a graph, a photograph, a list, various kinds of methods including the cartoon film, and it is not limited. For such a technology, for example, in METHODS IN CELL BIOLOGY, VOL.56, ed. 1,998, pp : In 185-215, A High - Resolusion Multimode Digital Microscope System (Sluder & Wolf, Salmon), the design of the hardware system is argued with applications software to automate a microscope or to control a camera,including automating a microscope, including the automatic light microscope, a camera, the Z-axis focus device and can use it in this invention. The imago acquisition with the camera is Inoue and Spring, Video Miroscopy, 2d. It is listed in Edition, 1997 in detail, and it is quoted for references in an instant specification.

For a mathematical treatment used in the specification, for example, the publicly-known technology described in Mathematics for Life System Analysis, Corona Publishing Co., Ltd., Kazuyuki Shimizu (1999) is applicable.

The model of the tertiary structure of the compound which there is expressed with a computer with "the tertiary structure (a computer) model" of the protein in an instant specification. Program, DENZO (HKL2000), MolScript (Avatar Software AB), Raster3D, PyMOL (DeLano Scientific), TURBO - FRODO(AFMB - CNRS), O (A.) which can display such a model with a well-known computer program in the field concerned, and, for example, are supported in CCP4 for such a program. There are Jones, Uppsala Universi tet, Sweden), ImageMagic (John Chrysty), RasMol (University of Massachusetts, Amherst MA USA), but it is not limited to them. Such a program can generate a model with the data of the atom coordinate.

"Pharmacophore" or "pharmacophore" in this specification is a combination with with an atom and the functional group (and those three dimensions-like locus), and a drug can interact with target protein by a specific method by using this and shows activity of the pharmacology. It is the three-dimensional "functional form" formed by the solid (of the physics) of molecule drug electric field, and activity of the pharmacology of the monad hereby produces it. Various approach method to study the measurable activity of a medical and pharmaceutical lead compound and the lead compound for the specific target is developed, and it can be designed the correlation of a series of conformation activity pharmacophore.

The screening of the pharmacophore (a carrier of the medicine) is technique taken for a routine in a computer-assisted medicine design; (P. W. Sprague et al., Perspectives in Drug Discovery and Design, ESCOM Science Publishers B. V., K. Muller, ed .1995,3,1; D. Barnum et al., J. Chem. Inf. Comput. Sci., 1996,36,563; J. Greene et al., J. Chem. Inf. Comput. Sci., 1994,34,1297 references. which quotes the above for references in an instant specification.) It is thought that the screening of the pharmacophore is effective for the analysis of many compounds given by high-throughput screening and combinatorial chemistry. The concept of the pharmacophore is based on interaction observed in molecular recognition such as a hydrogen bond and ionization, the hydrophobic symphysis. The pharmacophore is defined as ligand group as modality (for example, aromatic center, minus electric charge center, a hydrogen bond donor) of the functional group group in a specific conformation (for example, triangular) expressing common interaction between one biological target. In this definition, pharmacophore is a three-dimensional descriptor (a 3D descriptor).

For a commercial software system carrying out screening of the pharmacophore, for example, Catalyst (Molecular Simulations Inc.) 9685Scranton Road made, San Diego, CA92121-3752) (P. W. Sprague et al., Perspectives inDrug Discovery and Design, ESCOM Science Publishers B. V., K. Muller, ed.1995,3,1; D. Barnum et al., J. Chem. Inf. Comput. Sci., 1996,36,563; J. Greene etal., J. Chem. Inf. Comput. Sci., ChemDiverse module (ChemicalDesign Ltd.) of 1,994,34,1,297 references) and Chem - XProduct made in, Roundway House, Cromwell Park, Chipping Norton, Oxfordshire, OX75SSR, U. K). (S. D. Pickett et al., J. Chem. Inf. Comput. Sci., 1996,36,1214 references. quoting this for references in an instant specification is given.) But unfortunately the registration of the compound to the unsociable database system which a manufacturer owns is imposed duty on in the advantage of these software systems.

The pharmacophore finger printing expanded the above-mentioned approach to constitute a unit set of the pharmacophore by pharmacophore of various modality having various kinds of distance ranges. Applying a unit set of the pharmacophore to the compound group and generate the pharmacophore finger printing which is a descriptor showing important character in ligand - receptor symphysis. It is about pharmacophore finger printing. A.C. Good et al., J. Comput. Aided Mo. Des., 1995,9,373; J. S. Mason et al., Perspective in Drug Discovery and Design, 1997,7/8/,85; S. D. Pickett et al., J. Chem. Inf. Comput. Sci., 1998,38,144; S. D. Pickett etal. J. Chem. Inf. Comput. Sci., 1996,36,1214; C. M. Murray et al., J. Chem. Inf. Comput. Sci., 1999,39,46; J. S. Mason et al., J. Med. Chem. 1,999,39,46; S. D. Pickett etal. J. Chem. Inf. Comput. Sci., 1998,38,144; R. Nilakantan et al., J. Chem. Inf. Comput. Sci., It is explained in detail to 1993,33,79. In addition, about the application to a structure activity correlation, it is X. Chen et al., J. Chem. Inf. Comput. Sci., It is reported to 1998,38,1054. Quoting above-mentioned each for references in an instant specification.

The calculated molecular descriptor has some desired character. It is desirable for the descriptor to give a quantitative aim of the molecular similarity. The use of the molecular descriptor spreads by connecting it with measurable character experimentally. For example, it is enabled to bring logP(Here, logP means hydrophobicity of the material and transitional endpoint.) into operation value close to measurements as much as possible. The symphysis of the ligand for the biological target is important character in the medicine design. When the structure of the target cuts it in advantage (in, for example, using docking operation), the bind of the ligand can be calculated definitely. However, there is usually much circumstance estimating symphysis of the ligand from simpler calculation character to be able to consider to be an independent variable. The descriptor including the conformational information becomes the superior tool than estimation of bioactivation.

"Gene" in the specification means a factor to specify a genetic character. Genes are arranged on a chromosome in certain order. The gene for specifying a primary structure of protein is referred to as structural gene, and the gene affecting the manifestation is referred to as a regulatory gene. In the specification, under a specific situation, "gene" may indicate "polynucleotide", "oligonucleotide" and "nucleic acid" and/or "protein", "polypeptide", "oligopeptide" and "peptide".

The macromolecule conformation (for example, polypeptide conformation) can be described about the constitution of various kinds of levels. For example, about the general argument of this constitution, it is Alberts, Molecular Biology of the Cell (the third edition, 1994) and Cantor and Schimmel, Biophysical Chemistry Part I : refering to The Conformation of Biological Macromolecules (1980). "Primary structure" in the specification means an amino-acid sequence of specific peptide. "Secondary structure" means a three-dimensional structure locally arranged within polypeptide. These structures are generally publicly-known as domains. The domain forms a dense unit of polypeptide, and is mainly a portion of polypeptide with 50 to 350 amino acid length. The typical domain is $\beta$-sheer and $\alpha$-helix. "Tertiary structure" means a complete three-dimensional structure of polypeptide monomer. "Quaternary structure" means a three-dimensional structure formed by noncovalently-assembly of independent tertiary unit. The term about the anisotropy is used like term to be known in the field of energy.

The crystal structure analysis of the protein can be performed with a well-known method in the field concerned. For example, it is listed in a X-ray crystallographic analysis (Springer-Verlag Tokyo company) of the protein, an introduction to crystallographic analysis for life sciences (Maruzen), and such a method can use such a method with the instant specification voluntarily.

"Topology" in this specification means a secondary structure unit of the protein or space placement. About a tertiary structure, comparison with the tertiary structure of various kinds of protein (including the fragment of the protein which a function resembles) registered with a protein data bank (PDB) which is the public data bank of the protein tertiary structure can be performed from the viewpoint of topology.

"Tertiary structure data" or "the atom coordinate data" of the protein means the three-dimensional structure of the protein. Atom coordinate data, topology, a molecular force field fixed number are nominated for tertiary structure data of the protein typically. Atom coordinate data are data provided from a X-ray crystallographic analysis or NMR structure elucidation typically and such an atom coordinate data perform a X-ray crystallographic analysis or NMR structure elucidation newly and can be provided or can obtain it from the well-known database (for example, protein data bank (PDB)). Atom coordinate data can be data made again by modeling or a calculation.

The topology can be calculated with the tool program of a market or the freeware, but may be used one's own program. In addition, the molecular topological calculation program attached to a commercial molecular force field calculation program (the prepar program, for example, is attached to PRESTO, protein engineering research institute Co., Ltd.) can be used. The molecular force field fixed number (or molecular force field potential) can be calculated with the tool program of a market or the freeware again, too, and one's own data may be used. In addition, the molecular force field fixed number data which are attached to a commercial molecular force field calculation program (for example, AMBER, Oxford Molecular) can be used.

What kind of character (for example, catalytic activity, interaction with the other molecule) each amino acid carries by analyzing protein before the transmutation or the amino acid sequencing of the polypeptide monad (molecule wild type) and a tertiary structure, and, in an instant specification, the design of the change body monad is performed by calculating the amino acid transmutation that is appropriate to bring desired characteristic change (for example, improvement of the catalytic activity, improvement of the stability of the protein). Preferably the method of the design is taken with a computer. The following is given so that it is mentioned in an instant specification for an example of a computer program used by such a design method : DENZO (a Mac science) which is a handling of X-ray diffraction data program as a program to parse a conformation, As a processing program to decide a phase PHASES (Univ.) of Pennsylvania, PA, USA), As a program for initial phased improvement program DM (CCP4 package, SERC), As a program to get 3D-Graphics program O (Uppsala Universitet, Uppsala, Sweden), A tertiary structure is precise; as a program XPLOR (Yale University, CT, USA), And as a program for transmutation transduction modeling Swiss - PDBViewer (above mentioned).

It can be used in the process how the computer program to perform computer modeling chooses a fragment or a chemical compound again. The following is nominated for such a program.

1. GRID (P. J. Goodford, "A Computational Procedure for Determining Energetically Favorable Binding Sites on Biologically Important Macromolecules", J. Med. Chem., 28,849-857 pages of (1985)). GRID is available from Oxford University, Oxford, the UK.

2. MCSS (A. Miranker, "Functionality Maps of Binding Sites" : A Multiple Copy Simultaneous Search Method Proteins : Structure, Function and Genetics, 11,29-34 pages of (1991)). MCSS is available from Molecular Simulations, San Diego, CA.

3. AUTODOCK (D. S. Goodsell, "Automated Docking of Substrates to Proteins by Simulated Annealing", Proteins : Structure, Function, and Genetics, 8,195-202 pages of (1990)). AUTODOCK is available from Scripps Research Institute, La Jolla, CA.

4. DOCK (I. D. Kuntz, "A Geometric Approach"to Macromolecule - Ligand Interactions, J. Mol. Biol., 161,269-288 pages of (1982)). DOCK is available from University of California, San Francisco, CA.

"Library" in the specification is a certain assembly of chemical substances, such as compounds or organisms, for screening. The library may be an assembly of compounds having a similar nature or an assembly of random compounds. Preferably, an assembly of compounds, which expectedly have a similar nature, is used; however, the assembly is not limited to this.

"Interaction" in the specification means an action between one molecule and another molecule in the case that two or more molecules exist. As such interaction, hydrogen bond, Van der Waal's forces, ionic interaction, nonionic interaction, receptor ligand interaction, electrostatic interaction and host-guest interaction can be exemplified; however, the interaction is not limited to them. As one of techniques for quantifying the intermolecular interaction, thermodynamic or kinetic quantification evaluation method for intermolecular interaction can be exemplified; however, the technique is not limited to this. As the thermodynamic or kinetic quantification evaluation method for intermolecular interaction, for example, calorimetory, surface plasmon resonance method and ultracentrifugal analysis method can be exemplified; however, the method is not limited to these. The intermolecular interaction is expressible with an index indicating a change in thermodynamic quantity between a state where a complex is formed and another state where the complex is dissociated, and for example, it is expressible with a binding constant, a dissociation constant, a change in standard chemical potential associated with binding/dissociation, a change in enthalpy and a change in the number of ion bindings.

"Interaction information" in the specification is, for example, expressed with a presence of intermolecular binding, binding activity and pharmacological activity; however, this information is not limited to them. "Pharmacological activity" is an index indicating potency of drug in general. For example, it is provided as IC50, which is concentration indicating 50 % of inhibitory effect on physiological activity, or EC50, which is concentration showing 50 % of increase effect on physiological activity, and for "presence of binding" and "binding activity", for example, it is provided as a dissociation constant Kd.

"Information" in the specification, is the aggregation of facts or data having meaning in constant context.

"Data" in the specification are expressions of the information, and it is term of the public who shows formalization, an encoded thing to be suitable for interpretation, processing. For example, I show a number, an English letter, sign, analog quantity, a letter given meaning or an analog quantity expression when it is used for input of the computer processing. For example, in protein, I call data about the three-dimensional structure of the protein the "tertiary structure data" or "atom coordinate data".

"Information processing" or "processing" in the specification means to convert or integrate from one form to another form for the purpose of easily handing information. Further, "data processing" means a process to convert or integrate data from one form to another form.

"Pattern recognition" in the specification is one of natural information processing. For example, this is to identify and recognize information, which will not be provided as a simple quantity, such as mode, figure, object, image, sound or physiological phenomenon. Such information is referred to as "pattern information" or simply "pattern". As a classifier for conducting the pattern recognition, for example, it is possible to use a technique for comprising a classification parameter from a large quantity of data according to machine learning, such as a support vector machine (SVM), Bayesian classifier. "Interaction pattern" in the specification means information to be defined as a statistic model according to the classifier in the specification.

"Model" in the specification means a mathematical or physical system in accordance with a specific condition, and the model is used for understanding the system in the natural science, such as physics or biology. In particular, in the case of becoming a target for statistic analysis, the model is referred to as "statistic model". In the case that this is used in the specification, "modeling" of a phenomenon is to introduce "model" for elucidation, estimation and control of the phenomenon behind the data and discovery of new knowledge. In the specification, when an element in an assembly X is provided, the association with one element in an assembly Y by the function *f* is expressed as "mapping from the assembly X to the assembly Y". Further, the transition from the assembly X to the assembly Y by the function *f* is referred to as "map transformation". In the present invention, an assembly means an assembly of chemical substances. Further, "mapping" in the specification is to multiply test data by a calculated weighting factor matrix or a kernel function, by map transformation of training data with a calculation, such as CCA, PCA or becoming kernel.

The library in the specification can be produced or acquired by a technique, for example, a combinatorial chemistry technology, a fermentation procedure or plant and cell extraction procedure. A method to create the combinatorial library is publicly known in the technical field. For example, see E.R. Felder, Chimia, 1994, 48, 512-541; Gallop et al., J. Med. Chem. 1994, 37, 1233-1251; R.A. Houghten, Trends Genet. 1993, 9, 235-239; Houghten et al., Nature 1991, 354, 84-86; Lam et al., Nature 1991, 354, 82-84; Carell et al., Chem. Biol. 1995, 3, 171-183; Madden et al., Perspectives in Drug Discovery and Design2, 269-282; Cwirla et al., Biochemistry 1990, 87, 6378-6382; Brenner et al., Proc. Natl. Acad. Sci. USA 1992, 89, 5381-5383; Gordon et al., J. Med. Chem. 1994, 37, 1385-1401; Lebl et al., Biopolymers 1995, 37, 177-198; and references cited in these. The entire references are incorporated in the specification as reference.

Associated with the recent development of the combinatorial chemistry and the high throughput screening, it has become possible to experimentally approach many compounds. For example, see D.K. Agrafiotis et al., Molecular Divesity, 1999, 4, 1; U. Eichler et al., Drugs of the Future, 1999, 24, 177; A. K. Ghose et al., J. Comb. Chem., 1, 1999, 55; E. J. Martin et al., J. Comb. Chem., 1999, 1, 32; P. R. Menard et al., J. Chem. Inf. Comput. Sci., 1998, 38, 1204; R. A. Lewis et al., J. Chem. Inf. Comput. Sci., 1997, 37, 599; M. Hassan et al., Molecular Divesity, 1996, 2, 64; M. J. McGregor et al., J. Chem. Inf. Comput. Sci., 1999, 39, 569; R. D. Brown, Perspectives in Drug Discovery and Design, 1997, 7/8, 31. They are incorporated in the specification as references. Consequently, the technology to analyze a calculation characteristic regarding many compounds has become more and more important in the drug development. In two application examples of specific libraries, i.e., a structure of target library and a structure of primary library, information, which is especially important for a drug design, can be provided by the analysis of calculation characteristic regarding many compounds.

The construction of the target library developed calculation chemistry and the technology of the monad model to use a structure activity correlation (QSAR) of the fixed-quantity for a scafold (a three-dimensional conformation motif) design and the selection of the building block essentially. In QSAR, a molecular descriptor (a D scripter) is calculated, and with this molecular descriptor, a model expecting bioactivation for the individual target is built.

Generating an active compound for one or the plural targets without needing information about the conformation of a receptor (an acceptor) and the ligand (a thing connected to an acceptor) using a primary library can be performed. In addition, screening the primary library for unrelated various targets for many conformations can be performed. Furthermore, the elaboration of a compound having the most suitable absorption which is the character that is unrelated to the ligand symphysis which is the important activity of molecule medicine activity, distribution, metabolism, excretion (ADME) and a toxicity profile can use a primary library.

Furthermore, a middle library for the identification of the compound with the activity structurally for related compound group can be used. Therefore, the intermediate library has both the character that is characteristic of two of a target library and the primary library.

(Application of this invention)

In the present invention, the compound space (in other words chemical space) and the biological substance space (in other words biospace) as a model is explained below.

Coordinate space showing the relative locus correlation of the individual compound is necessary for the way of basic thinking as performing a lead search and the library design of the compound by a calculation. For example, the coins on the chemical space of Figure 1 to 3 expresses a different compound each. The compounds which have similar character is positioned, and the coordinate space which is consistant of positions of these compounds is called chemical space. In the same way, it is bio space to express analogous relationship of gene (protein) as a relative postion (the lower stage, a square mark expresses gene or protein). Moreover the model of integration between chemical space and bio space is able to be made by linking each compound to the protein that the compounds can combine.

When this model of integration between chemical space and bio space exists, and target protein for a compound whose activity is unknown is estimated from the integrated model of the chemical space and the biological space, 1) the compound whose activity is unknown is mapped to the chemical space coordinate according to the chemical structure of the compound (star mark) 2) By tracing the link information from the compound adjacent to the compound mapped to the chemical space to the biological space, an area (within the circle in lower stage of Fig. 2) within the biological space where the unknown compound is related can be specified. 3) The protein group within this area is assumed as a protein group where this compound with unknown activity is possibly interacted (Figure 2).

In addition, the prediction of a lead compound acting on a disease target gene is enabled if there is this " integrated model of chemical space and biological space ", for example, to show it with the arrow of the figure (figure 3).

Furthermore, the integrated model of chemical space and biological space is applicable to a design of the efficiency of the compound library (figure 4).

As the figure indicates, compounds in the area which correspond to the yellow bio space (biologically relevant chemical space) are thought to have possibilities of interacting with protein group which consist bio space. Thus, the compound library with biological activity is able to be designed.

Further, if protein in the biological space is, for example, limited to GPCR family, a focused library can be designed.

(A definition of the space)

The feature of this technique is indicated about the organization method of the most important "the integrated model of chemical space and biological space ".

The prior technique (the upper section) defined coordinates of chemical space, using only chemical information so that the chemical characters of the compound become various as possible.

Here, the big problem is that there is no causal association between the variation of compounds and biological activities.

Therefore this technique uses both the chemical information and protein arrangement information, and defines each other's coordinates in space so that a correlation of both space of chemical space and the protein space rose. If the chemical space coordinates are difined with consideration of relevance with biological space, space coordinates which is more convenient for biological activity can be built.

In-silico screening was conducted using "integrated model of chemical space and biological space", in order to evaluate the performance of the conventional method (PCA) and the present technique (CCA), constructed by both methods, respectively.

Figure 6 shows a ROC curve, which is one of the methods to evaluate the estimation performance. This graph indicates that the more upper the curve is positioned, the better the estimation performance is, and since the curve of the present technique is positioned more upper than that of the conventional method, the estimation performance of the present technique is higher than that of the conventional method.

In figure 7A to 7C, one embodiment of the compound - GPCR interaction prediction technique is showed. SVM method in this invention was applied to estimation for interaction between G Protein Coupled Receptor and compounds, and the evaluation of estimation was performed. The result of the evaluation of estimation performance, a 5-fold cross validation said that it rightly predicted interaction of about 82% by the prior method based on a compound conformation, about 91 % by SVM method based on interaction information.

Furthermore, ligand estimation of human β2 adrenaline receptor (β2AR) was performed by SVM method, and inspected it by in vitro experiment and so on. 81% (17/21) of estimated ligands are found to actually bind, and detection of the β2AR ligand which had new structure which was not found by the prior method was succeeded.

These results show that Chemicalgenomix information is useful for not only the improvement of the ligand prediction precision but also detection of ligand having new structure.

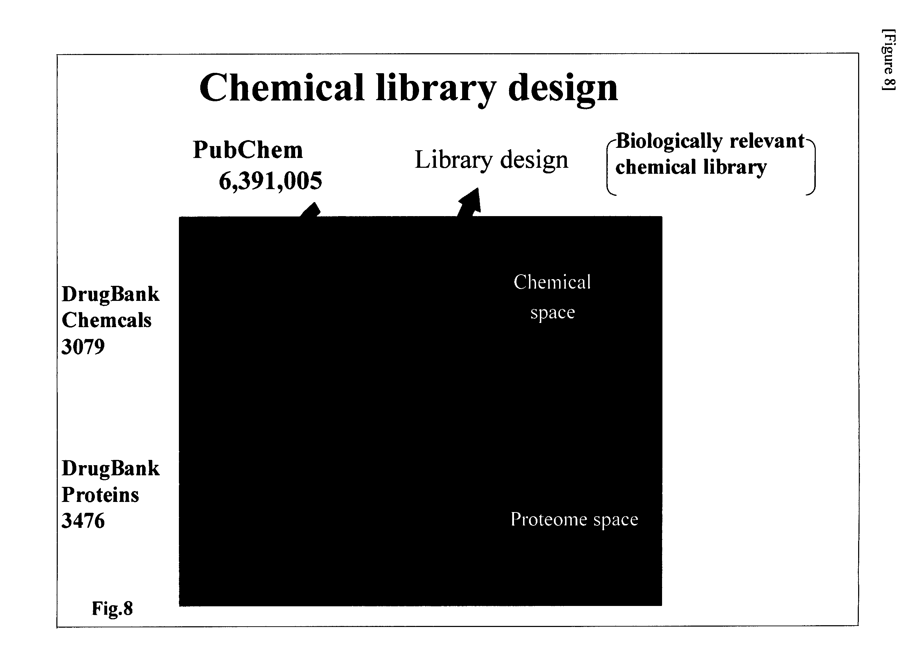

As figure 8 is shown, this technique was actually used to design the compound library (the prediction of the target gene), because the performance of the "in silico screening" prediction with this technique was better than one with prior art.

Protein candidates, which can be targeted by these compounds, were estimated using 6,391,005 compounds within the US NCBI/PubChem database, and a compound library was constructed. Herein, for the preparation of the integrated model between the chemical space and the biological space, which are reference coordinates for estimation, Canada's DrugBank database where data about drugs and target proteins thereof are accumulated was used.

Fig. 9 shows results of bioactivity estimation of PubChem compounds. Each row shows data per score indicating the reliability of binding possibility between a compound and a target protein. In other words, it appears that the higher the score becomes, the higher the reliability of the bioactivity of the compound. It appears that the score herein shows a bindability (statistic significance of binding estimation) between any chemical compound A within the first space and any chemical substance B within the second space.For example, the binding score between the chemical substance A and the chemical substance B can be defined as follows: It is assumed that the number of all chemical substances within the second space is N, and the number of substances to be bound with the chemical substance A is L. Herein, in the case of considering K chemical substances within the second space adjacent to B, it is assumed that the number to be bound with the chemical substance A is H. On that occasion, the binding score from the chemical substance A to B can be defined as odd score, such as $\log(H/K)/(L/N)$. Inversely, it is assumed that the number of all chemical substances within the second space is n, and the number of substances to be bound with the chemical substance B is $l$. Herein, in the case of considering $k$ chemical substances within the first space adjacent to A, it is assumed that the number to be bound with the chemical substance B is $h$. On that occasion, the binding score from the chemical substance B to A can be defined as an odd score, such as $\log(h/k)/(l/n)$. In Fig. 9 the overall score of the binding possibility between the chemical substances A and B were defined as $\log((H/K)/(L/N)) + \log((h/k)/(l/n)) + 20$ from the score from the chemical substances A to B and the scores from B to A.

Further, the items in each line indicate the classification per function of the target protein (based upon gene ontology). The numerical values in the table represent the number of (estimated) compounds corresponding to the correspondence portion. For example, protein regarding the receptor activity is targeted, and for compounds showing score value: 27 or more reliability, 198 compounds are estimated.

Fig. 10, as similar to Fig. 9, shows results of bioactivity estimation of PubChem compounds, and the function of the target protein is classified using different references, respectively. The view of Fig. 10 is similar to Fig. 9.

Hereafter, explanation of an embodiment for implementing the present invention is described; however, this embodiment is simply an exemption for implementing the present invention, and it should be understood that the scope of the present invention shall not be limited to such preferred embodiment.

In one enbodyment, this invention provides a data structure which defines the fisrt space expressing coordinates of the first chemical substance group and the second space expressing coordinates of the second chemical substance group, and the first chemical substance group is characterized by the first characteristic amount, and the second chemical substance group is characterized by the second characteristic amount. Here, the data structure is the logical conformation that expressing mutual relationship between the data elements.

In one enbodyment, simple relevancy cannot be confirmed between the first characteristic amount used in this invention and the second characteristic amount used in this invention.

For example, the quantity of first character is chemistry character of the first chemical substance, and, for a certain concrete enforcement form, the characteristic amount of the second above can be biological activity of the second chemical substance. Biological activity and the chemical activity are the representatives of the characteristic group where simple relevancy is not found in each other.

In another embodiment, the first chemical substance is a compound, and the second chemical substance is biological substance. In this case the first space is called chemical space, and the second space can be called biospace. As the compound, any compound library can be used. For example, a combinatorial library, any compound library owned by a corporation, database managed by a public agency, compound library database managed by consortium can be exemplified; however, the compounds are not limited to those. For example, the above biological substance can be a nucleic acid, peptide or polypeptide or protein, saccharide or polysaccharide, lipid and those complexes, but, for biological substance, it is not limited to them.

Any kind of information for the making of coordinates in space of this invention can be used. For example, in the case of coordinates in space of the biological substance, I can use at least one kind of information chosen from the arrangement information, secondary structure, tertiary structure, quaternary structure, tertiary structure information, expression information, pass way information and function information group.

In a preferred embodiment, a coordinate of the first space and a coordinate of the second space are defined so that the correlation between the first space and the second space becomes maximum. Herein, the correlation between the first space and the second space can be increased by a multivariate analysis technique, such as canonical correlation analysis (CCA), kernel canonical correlation analysis (kernel CCA) or a support vector machine (SVM) method, or a machine learning method or an equivalence method thereof. The protocol of CCA, SVM and etc itself can be used.

Here, correlated between the first space and the second space is performed by, for example, a canonical correlational analysis (CCA), a kernel canonical correlational analysis (kernel CCA), support vector machine (SVM), multivariate analysis technique, a mechanic learning method or an equivalence of value method.

The canonical correlation analysis (CCA) is a type of the multivariate analysis technique for analyzing the correlation between data sets when two types of foreign data sets, for example, a compound and protein, are provided. CCA is to conduct the map transformation so as to express the correlation between both data sets the best, and then, this result in the analysis of the correlation between the two data. Specific procedures are shown below.

For the specific procedures of CCA, the following can be exemplified: (T. W. Anderson, An Introduction to Multivariate Statistical Analysis, Wiley & Sons, 1984, H. Hotelling, Relations between two sets of variates, Biometrika, 28: 321-377, 1936).

When two types of foreign data (for example, a compound and protein) where an entry of e chemical substance are aligned in a row and chemical substance information is aligned in a column are expressed as matrixes X and Y (the first space is a matrix X and the second space is a matrix Y),

[Number 111]

$$X = n \times p \qquad Y = n \times q \qquad n \geq p \geq q \geq 1$$

$$f = Xa \qquad g = Yb$$

$$\sigma_f{}^2 = \frac{1}{n-1} f'f \qquad \sigma_{fg} = \frac{1}{n-1} f'g \qquad \sigma_g{}^2 = \frac{1}{n-1} g'g$$

in order to maximize the correlation between the first space and the second space, a set of coefficient vectors a and b to maximize the correlation is searched, and

[Number 112]

$$r_{fg} = \frac{\sigma_{fg}}{\sigma_f \sigma_g}$$

herein,
**[0129]**

[Number 113]

$$\sigma_f{}^2 = \sigma_g{}^2 = 1$$

with the following conditions:
[Number 114]

$$r_{fg} = \sigma_{fg}$$

is maximized,
**[0133]**

[Number 115]

$$r_i$$

is referred to as canonical correlation, and
[Number 116]

$$f, g$$

To be more specific, in the canonical correlation analysis, a singular value decomposition of X and Y is conducted,
**[0137]**

[Number 117]

$$X = U_X D_X V'_X \qquad Y = U_Y D_Y V'_Y$$

$$U'_X U_Y = UDV'$$

U, D and V are calculated, and using U, D and V, and the following are acquired:
[Number 118]

$$A = \sqrt{n-1}\, V_X D_X^{-1} U \qquad B = \sqrt{n-1}\, V_Y D_Y^{-1} V$$

$$F = \sqrt{n-1}\, U_X U \qquad G = \sqrt{n-1}\, U_Y V$$

it is provided that A, B, F and G are as follows:
[Number 119]

$$f_i = Xa_i \qquad g_i = Yb_i \qquad i = 1, \ldots, q$$

$$A = [a_1, \ldots, a_q] \qquad B = [b_1, \ldots, b_q]$$

$$F = [f_1, \ldots, f_q] \qquad G = [g_1, \ldots, g_q]$$

$$F = XA \qquad G = YB$$

herein, the higher correlation can be obtained from i=1 in order

[Number 120]

$$r_{fg}$$

is possible to get

The kernel canonical correlation analysis (kernel CCA) is a technique introducing a kernel method to a normal canonical correlation analysis, and with regard to the canonical correlation analysis based upon a linear model, a correlation analysis based upon a nonlinear model is possible with the kernel canonical correlation analysis.

This is a method to conduct the canonical correlation analysis to X' and Y' where X in the first space and Y in the second space, which were targets in the canonical correlation analysis (S. Akaho, A kernel method for canonical correlation analysis, International Meeting of Phychometric Society (IMPS), 2001).

The support vector machine (SVM) method is a machine learning algorithm for solving a supervised identification problem (reference: B. E. Boser, I. M. Guyon, and V. N. Vapnik, A training algorithm for optimal margin classifiers, In D. Haussler, editor, 5th Annual ACM Workshop on COLT, pages 144-152). In SVM, it is characterized such that the way of thinking to maximize a margin for obtaining a separated plane (hyperplane) where the distance from each data point becomes maximized in order to classify data into two types is used. In addition, it is also characterized such that a superior performance even in the non-linear separation problem is confirmed by using a method where a pattern is map-transformed to limited or infinite dimensional feature space using the kernel function. Herein, when a supervised classification problem is applied to a binding estimation of protein and a compound, it results in the creation of a classifier to classify a class representing binding between the protein and the compound and another class representing not binding. In this case, known binding data between protein and a compound, which can be obtained from references or experiments, can be used as supervision data.

The multivariable analysis technique is a generic term of statistic technique to be used for acquiring a mutual relationship between variables by utilizing data (multivariable data) having a plurality of variables (item, attribute and number of dimension). Techniques, such as multiple regression analysis, discrimination analysis, canonical analysis, principal component-factor analysis, cluster analysis, multidimensional scaling, face analysis, quantification analysis, or conjoint analysis, are available. These are effective to "summarize" tendency and/or characteristic in complicated data, for "discovery of a cause" or "presumption/ estimation" by clarifying a correlation that affect results, or modeling of cause-effect relationship. CCA and kernel CCA also correspond to this multivariable analysis.

The machine learning approach is one of research projects in artificial intelligence, and is a technology/technique for realizing a function, which is similar to learning capability naturally performed by a human. A sample data assembly with a certain number of samples as targets is analyzed, and useful regulations, rules, knowledge expression and judgmental standard are extracted from the data.

They can be put together as follows.

In one enbodiment, the coordinates in space of the first chemical substance in this invention are defined by chemical information. Here, the chemical information is defined by a compound descriptor.

In a concrete embodiment, the compound descriptor is chosen from the one-dimensional descriptor, two-dimensional descriptor and three-dimensional descriptor group. In one embodiment, a compound descriptor is a one-dimensional descriptor, and it is character of this one dimension descriptor that describes chemical composition.

In another embodiment, a compound descriptor is a two-dimensional descriptor, and it is character of this two dimension descriptor that describes chemical topology.

In another embodiment, a compound descriptor is a three-dimensional descriptor, and it is character of this three-dimension descriptor that describes character chosen from the three-dimension configuration and functional group.

In another embodiment, a compound descriptor is pharmacophore.

In a concrete embodiment, the pharmacophore includes at least three spatially remote pharmacophore center, and each pharmacophore center includes (i) space locus and (ii) appointed pharmacophore which identify a chemical character, and the basic set of pharmacophore includes, at least, a hydrogen bond acceptor, a hydrogen bond donor, negative charge center, positive charge center, hydrophobicity center, aromatic series center and the default category which does not belong to any other pharmacophore model.

In one embodiment, pharmacophore can be described in more detail by providing with a spatial distance as an isolated destance between adjacent pharmacophores or an isolated distance range..

(Production procedure)

In one situation, this invention offers a method to produce chemical compounds having desired character. This method includes A) a process of offering the first chemical substance group included in the first space defined by the database of coordinates of the first chemical substances, B) a process of offering the second chemical substance group included in the second space defined by the database of coordinates of the second chemical substances, and the first chemical substance is characterized by the first characteristic amount, and the second chemical substance is characterized by the second characteristic amount, C) a process of choosing desired character of the second characteristic amount, D) a process of calculating a focused region in the second space of the second chemical substance having the said chosen desired character, E) a process of calculating a target region of the first space that is less than predetermined distance from the said focused region, and F) a process of choosing the chemical substances which is in the target region of the first said space.

"The focused region", used in this method, means a region in the focused space of the reference side of the screening (the query side).

"The target region" used in this method, means a region in the space of subjects which should be calculated in space of the subject side of screening (the output side).

Here, in A) a process of offering the first chemical substance group included in the first space defined by the database of coordinates of the first chemical substances, the chemical substances are described as chemical strucuter itself, various descriptors which is caliculated by computing from chemical structure, chemical characters which is estimated by computing from chemical strucutere or descriptors, or sequence (vector) which has numbers of chemical character gained by measuring compounds as elements. Thus, a position of each chemical substance is identified as vector in the coordinates of the first space.

In B) a process of offering the second chemical substance group included in the second space defined by the database of coordinates of the second chemical substances, the chemical substances are described as sequence (vector) which has numbers converted from bio information, such as arrangement information, a secondary structure, a tertiary structure, a quaternary structure, tertiary structure information, expression information, pass way information, function information and the biological activity information, with computing process or mearsuring. Thus, a position of each chemical substance is identified as vector in the coordinates of the second space.

Here, the first chemical substance is characterized by first characteristic amount, and the second chemical substance is characterized by the second characteristic amount.

Preferably, there is no simply mutually relationship between the first characteristic amount and the second characteristic amount. It is advantageous. It was found that the screening can be performed in more detail by usinig two more spaces defined by two such characters that are not relevant each other.

C) In a process choosing desired character in the second characteristic amount, the arbitrary desired activity that screening intends for can be choosen.

D) In a process of calculating a focused region in the second space of the second chemical substance having chosen desired character, the focused region can decide an arbitrary region in the space corresponding to the choosen character that is such as the activity above a certain level, for example.

In case that a specific protein (gene) or a protein group which is target in the screening, protein group which have homologous sequence or structure is selected, and the target region can be defined as the space region which the protein group occupy.

In addition, in case that the target region should be defined based on functions of proteins (for example, gene ontology), the protein group which is defined as that it has equivalent function is selected, and the target region can be defined as the space region which the protein group occupy.

Besides, in case that the target region should be defined based on a gene expression pattern, passway positional information or biological activity information (for example, microarray data, reaction course, pharamocology activity) that the protein has, the protein group which is defined as that it has equivalent gene expression pattern, passway positional information or biological activity information is selected, and the target region can be defined as the space region which the protein group occupy.

E) in a process of calculating focused region in the first space, when the focused region is fixed, those skilled in the art can caliculate the traget region which is in lower than appointed distance.

Compounds in the first space which can bind to each protein in the target region are identified. The focused region is calculated as the region in less than predetermined distance from identified compounds in the frist space. Here, the distance includes the similarity endpoint such as coefficient of correlation or the kernel from a thing meeting an axiom of distance such as Euclid distance, the Manhattan distance.

F) In a process of choosing the chemical substances which are in the focused region in the said first space, the chemical substances corresponding to the caliculated target region can be choosen. Here, once the space is defined, it is possible to choosing with automatic caliculation.

In one embodiment, this invention includes training process to let above the first space and the second space relate to with sample data.

"Training" in the specification is computer operation to need for training for use of the devices and is a console operation, a conversion operation, the activity that seem to be a print operation and the operation which is used though demonstrating.

"Training data" in the specification is the practice data which is input into a computer of robot at the beginning of the operation.

In one embodiment, the training generates an orthogonal matrix $A=C_{xx}^{-1/2}U$ and $B=C_{yy}^{-1/2}V$ (herein, det(A) = det(B) = 1 and as shown in the following mathematical expression):

[Number 131]

$$C_{xx}=E\{(X-m_x)(X-m_x)^T\}, \quad C_{yy}=E\{(Y-m_y)(Y-m_y)^T\}, \quad C_{xy}=E\{(X-m_x)(Y-m_y)^T\}, \quad K=C_{xx}^{-1/2}\cdot$$
$$C_{xy}\cdot C_{yy}^{-1/2}=U\cdot S\cdot V^T$$

It can be characterized such that the correlation between AX representing the first space of a first modality and BY representing the second space of a second modality becomes maximized, and due to this, it becomes possible to transfer from the first modality to the second modality.

In another embodiment, a matrix A and a matrix B are created according to the training. The correlation between XA representing the first space of the first modality and YB representing the first space of the second modality becomes maximized, and due to this, it becomes possible to transfer from the first modality to the second modality. For the transfer of characteristic, two types of foreign data (for example, a compound and protein) where entries of chemical substance are aligned in the row and chemical substance information is aligned in the column of the matrixes X and Y are expressed as matrixes X and Y (the first space is a matrix X and the second space is the matrix Y),

[Number 141]

$$X = n \times p \qquad Y = n \times q \qquad n \geq p \geq q \geq 1$$

$$f = Xa \qquad g = Yb$$

$$\sigma_f^{\,2} = \frac{1}{n-1} f'f \qquad \sigma_{fg} = \frac{1}{n-1} f'g \qquad \sigma_g^{\,2} = \frac{1}{n-1} g'g$$

in order to maximize the correlation between the first space and the second space, a set of coefficient vectors a and b to maximize the correlation is searched, and
[Number 142]

$$r_{fg} = \frac{\sigma_{fg}}{\sigma_f \sigma_g}$$

herein,
**[0179]**

[Number 143]

$$\sigma_f^{\,2} = \sigma_g^{\,2} = 1$$

with the following conditions:
[Number 144]

$$r_{fg} = \sigma_{fg}$$

is maximized,
**[0183]**

[Number 145]

$$r_{f,g}$$

is referred to as canonical correlation, and
[Number 146]

$$f, g$$

It is a plus associate variable
To be more specific, in the canonical correlation analysis, a singular value decomposition of X and Y is conducted,
**[0188]**

[Number 147]

$$X = U_X D_X V'_X \qquad Y = U_Y D_Y V'_Y$$

$$U'_X U_Y = UDV'$$

U, D and V are calculated, and using U, D and V, and the following are acquired:
[Number 148]

$$A = \sqrt{n-1} V_X D_X^{-1} U \qquad B = \sqrt{n-1} V_Y D_Y^{-1} V$$

$$F = \sqrt{n-1} U_X U \qquad G = \sqrt{n-1} U_Y V$$

it is provided that A, B, F and G are as follows:
[Number 149]

$$f_i = Xa_i \qquad g_i = Yb_i \qquad i = 1, \ldots, q$$

$$A = \lfloor a_1, \ldots, a_q \rfloor \qquad B = \lfloor b_1, \ldots, b_q \rfloor$$

$$F = \lfloor f_1, \ldots, f_q \rfloor \qquad G = \lfloor g_1, \ldots, g_q \rfloor$$

$$F = XA \qquad G = YB$$

herein, the higher correlation can be obtained from i=1 in order
[Number 150]

$$r_{fg}$$

It is get it

"Modality" in the specification means a characteristic attribute

In one embodiment, if only the result of the query of BY representing the second space, since BY has the maximum correlation with AX, it is possible to specify the query of AX representing the first space.

In the preferred embodiment of the invention, the process of the present invention includes that process A) - F) is perfomed by a mechanic learning method automatically. Any mechanic learning method can be used, but preferably the mechanic learning method is achieved by SVM method.

The support vector machine (SVM) method is a machine learning algorithm for solving a supervised identification problem (reference: B. E. Boser, I. M. Guyon, and V. N. Vapnik, A training algorithm for optimal margin classifiers, In D. Haussler, editor, 5th Annual ACM Workshop on COLT, pages 144-152). In SVM, it is characterized such that the way of thinking to maximize a margin for obtaining a separated plane (hyperplane) where the distance from each data point becomes maximized in order to classify data into two types is used. In addition, it is also characterized such that a superior performance even in the non-linear separation problem is confirmed by using a method where a pattern is map-transformed to limited or infinite dimensional feature space using the kernel function. Herein, when a supervised classification problem is applied to a binding estimation of protein and a compound, it results in the creation of a classifier to classify a class representing binding between the protein and the compound and another class representing not binding. In this case, known binding data between protein and a compound, which can be obtained from references or experiments, can be used as supervision data.

In the concrete embodiment, by the machine learning method, G1) a process of mapping query pair of the query first chemical substances and the second chemical substances into the space model which is built by the database of the coordinates in the first chemical substances and the database of the coordinates in the chemical substances; G2) a process of determine that the first chemical substance and the second chemical substance can bind each other if the said query pair is in the space region, of determine that the first chemical substance and the second chemical substance can not bind each other if the said query pair is not in the space region, are included.

In one important embodiment, there is not simple relevancy between the first characteristic amount and the second characteristic amount.

In another embodiment, the first characteristic amount is chemical character of the first chemical substance, and the second characteristic amount is biological activity of the second chemical substance. The example of no simple relevancy between the frist and the second characteristic amount is a pair of chemical character and biological character. Thus, in this case, the frist chemical substace is compound and the second chemical substance is biological substance.

Any biological substance can be used in this invention. For example, the above biological substance can be a nucleic acid, peptide or polypeptide or protein, saccharide or polysaccharide, lipid and those complexes. Therefore, the coordinates in space of the biological substance are defined by at least one kind of information chosen from the arrangement information, secondary structure, tertiary structure, quaternary structure, tertiary structure information, expression information, pass way information and function information group. Such informaition can be acquired from various kinds of databases and can be acquired from the database generated originally.

In the method of the present invention, the first above coordinate and the second above coordinate are defined as the correlation between first space and the second space becomes maximum, and a canonical correlational analysis (CCA), a kernel canonical correlational analysis (kernel CCA), support vector machine (SVM), multivariate analysis technique, a mechanic learning method or an equivalence of value method can be used. In addition, two of a canonical correlational analysis (CCA), a kernel canonical correlational analysis (kernel CCA), multivariate analysis technique including support vector machine (SVM) law, a mechanic learning method or equivalence of value methods may be applied together. The multivariate analysis technique can be a canonical correlational analysis (CCA) and a kernel canonical correlational analysis (kernel CCA). The mechanic learning method can include support vector machine (SVM).

In the invention, a correlation for target may be what kind of characteristic correlations, and preferably a correlation is a correlation between a focused region and the target region.

In one embodiment, the method of this invention includes a process of producing chemical compounds chosen more in silico. The production procedure in silico is listed in an instant specification in another place and can use well-known technology.

In another embodiment, the method of this invention includes a process producing chosen chemical compounds in wet. The wet production procedure is listed in another place of this specification and well-known technology can be used. As the exapmle of the wet production, it is possible to use combinatorial chemistry.

Or the wet production may be achieved with gene-recombination technology.

In one concrete embodiment, in addition, the method of this invention includes a process of selecting the chemical substances which have really desired activity, after choosing the above first chemical substances and determine the said second characteristic amount of the chemical substances in the said first space.

In one embodiment, in the process of selecting the chemical substances of this invention, the said chemical substances are scored based on the above second characteristic amount.

It appears that the score herein shows a bindability (statistic significance of binding estimation) between any chemical compound A within the first space and any chemical substance B within the second space.For example, the binding score between the chemical substance A and the chemical substance B can be defined as follows: It is assumed that the number of all chemical substances within the second space is N, and the number of substances to be bound with the chemical substance A is L. Herein, in the case of considering K chemical substances within the second space adjacent to B, it is assumed that the number to be bound with the chemical substance A is H. On that occasion, the binding score from the chemical substance A to B can be defined as odd score, such as $\log (H/K)/(L/N)$. Inversely, it is assumed that the number of all chemical substances within the second space is n, and the number of substances to be bound with the chemical substance B is $l$. Herein, in the case of considering k chemical substances within the first space adjacent to A, it is assumed that the number to be bound with the chemical substance B is $h$. On that occasion, the binding score from the chemical substance B to A can be defined as an odd score, such as $\log (h/k)/(l/n)$. In Fig. 9 the overall score of the binding possibility between the chemical substances A and B were defined as $\log ((H/K)/(L/N)) + \log ((h/k)/(l/n)) + 20$ from the score from the chemical substances A to B and the scores from B to A. Further, in the case of using the SVM method, the score can be converted from the equivalence of a distance from a hyperplane for separating a known binding pair and a not-binding pair between the chemical substance group within the first space and the chemical substance group within the second space.

The way of the description of a chemical compound and the biological substance, can be used by an arbitrary description method listed in another place of this specification.

In another situation, this invention offers a chemical compound produced by the process of the present invention.

In another situation, this invention offers a method to produce compound libraries. The production procedure of a compound library includes A) a process of offering the first chemical substance group included in the first space defined by the database of coordinates of the first chemical substances, B) a process of offering the second chemical substance group included in the second space defined by the database of coordinates of the second chemical substances, and the first chemical substances are characterised by the first characteristic amount, and the second chemical substances are characterised by the second characteristic amount, C) a process of choosing the desired character of the second characteristic amount, D) a process of caliculating the focused region of the second chemical subsatnces which have the said choosen desired character, in the second space, E) a process of E) a process of calculating a target region of the first space that is less than predetermined distance from the said focused region, F) a process of designing a library which has the desired character, by choosing two more chemical substances which is in the target region in the first space.

In the method of designing a library in this invention, any arbitary embodiment listed in the specification can be used.

In another situation, this invention offers a library designed by the process of this invention.

In another situation, this invention offers a program to let a computer carry out a method to produce chemical compounds having desired character. The method that this program has, A) a process of offering the first chemical substance group included in the first space defined by the database of coordinates of the first chemical substances, B) a process of offering the second chemical substance group included in the second space defined by the database of coordinates of the second chemical substances, and the first chemical substances are characterised by the first characteristic amount, and the second chemical substances are characterised by the second characteristic amount, C) a process of choosing the desired character of the second characteristic amount, D) a process of caliculating the focused region of the second chemical subsatnces which have the said choosen desired character, in the second space, E) a process of calculating a target region of the first space that is less than predetermined distance from the said focused region, F) a process of choosing two more chemical substances which is in the target region in the first space. The method of implementation by letting a computer implement the recording medium that the computer reading that a program was stored is possible through tool (if, for example, it is CD - R, CD-R drive) reading a recording medium. The description of the program can give any language (for example, C + language, perl, Basic, html, XML, Pascal, FORTRAN) in the field concerned, but it is not limited to them. In addition, it is understood that it points at a computer program at time called the program with the instant specification unless it is limited especially.

In another situation, the computer reading that stored a program to let a computer carry out a method to produce chemical compounds having desired character as for this invention offers a possible recording medium. The method that the program that this recording medium records carries out: A) a process of offering the first chemical substance group included in the first space defined by the database of coordinates of the first chemical substances, B) a process of offering the second chemical substance group included in the second space defined by the database of coordinates of the second chemical substances, and the first chemical substances are characterised by the first characteristic amount, and the second chemical substances are characterised by the second characteristic amount, C) a process of choosing the desired character of the second characteristic amount, D) a process of caliculating the focused region of the second chemical subsatnces which have the said choosen desired character, in the second space, E) a process of E) a process of calculating a target region of the first space that is less than predetermined distance from the said focused region, F) a process of choosing two more chemical substances which is in the target region in the first space. It is understood that any form (the arbitrary type that, for example, seems to be a flexible disk, MO, a CD-ROM, CD - R, a DVD-ROM) can be used as far as it can record a program for a recording medium.

This invention offers the recording medium which recorded data structure of this invention again. This recording medium is data structure possessing the second space defined by the first space defined by the database of the coordinates in space of the first chemical substance and database of the coordinates in space of the second chemical substance, and the first chemical substance is characterized by the first characteristic amount typically, and, as for the second chemical substance, data structure characterized by the second characteristic amount is recorded.

In another situation, this invention offers a system producing chemical compounds having desired character. This system :

A) a process of offering the first chemical substance group included in the first space defined by the database of coordinates of the first chemical substances, B) a process of offering the second chemical substance group included in the second space defined by the database of coordinates of the second chemical substances, and the first chemical substances are characterised by the first characteristic amount, and the second chemical substances are characterised by the second characteristic amount, C) a process of choosing the desired character of the second characteristic amount, D) a process of caliculating the focused region of the second chemical subsatnces which have the said choosen desired character, in the second space, E) a process of E) a process of calculating a target region of the first space that is less than predetermined distance from the said focused region, F) a process of choosing chemical substances which is in the target region in the first space, G) a process of producing the said choose chemical substances. For tool of A) - G) used here, it is understood that any embodiment listed in the specification can be used

In another situation, this invention offers a system to screen a chemical compound having desired character. This system includes, A) a process of offering the first chemical substance group included in the first space defined by the database of coordinates of the first chemical substances, B) a process of offering the second chemical substance group included in the second space defined by the database of coordinates of the second chemical substances, and the first chemical substances are characterised by the first characteristic amount, and the second chemical substances are characterised by the second characteristic amount, C) a process of choosing the desired character of the second characteristic amount, D) a process of caliculating the focused region of the second chemical subsatnces which have the said choosen desired character, in the second space, E) a process of E) a process of calculating a target region of the first space that is less than predetermined distance from the said focused region, F) a process of designing a library which has the desired character, by choosing two more chemical substances which is in the target region in the first space. For tool of A) - F) used here, it is understood that any embodiment listed in the specification can used.

In another situation, this invention offers a method to make a chemical compound library. This method includes A) the first space defined by the database of the coordinates in space of the first chemical compound, B) a process of when the character of one identifies the second space defined by the database of the coordinates in space of the known second chemical compound, and the first chemical substance is characterized by the first characteristic amount here, and, in the process when C) defines a coordinate of the first said space as for the second chemical substance again in a process characterized by the second characteristic amount so that a correlation with the first said space and the second said space becomes greatest, D) includes a process generating the first said space defined again as a new chemical compound library.

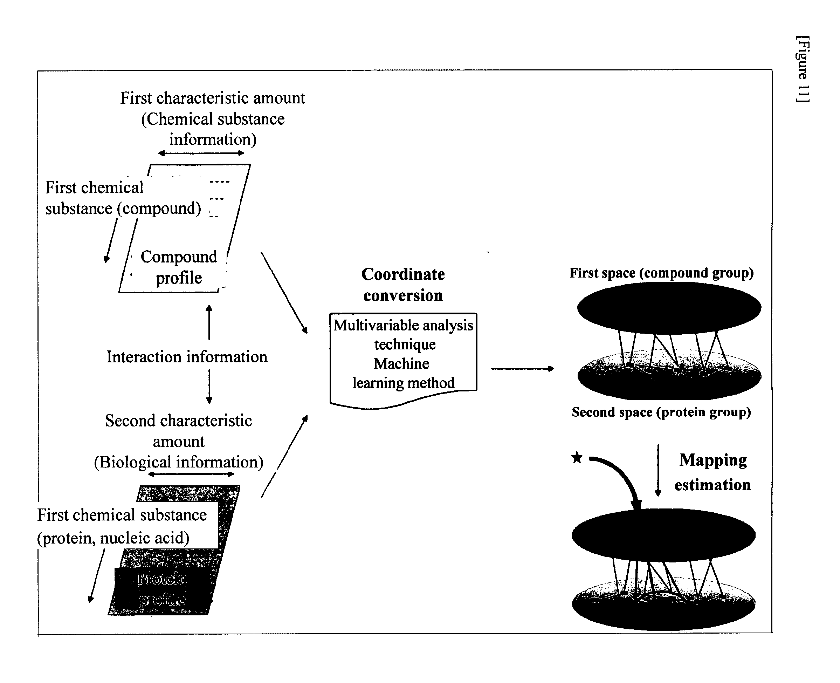

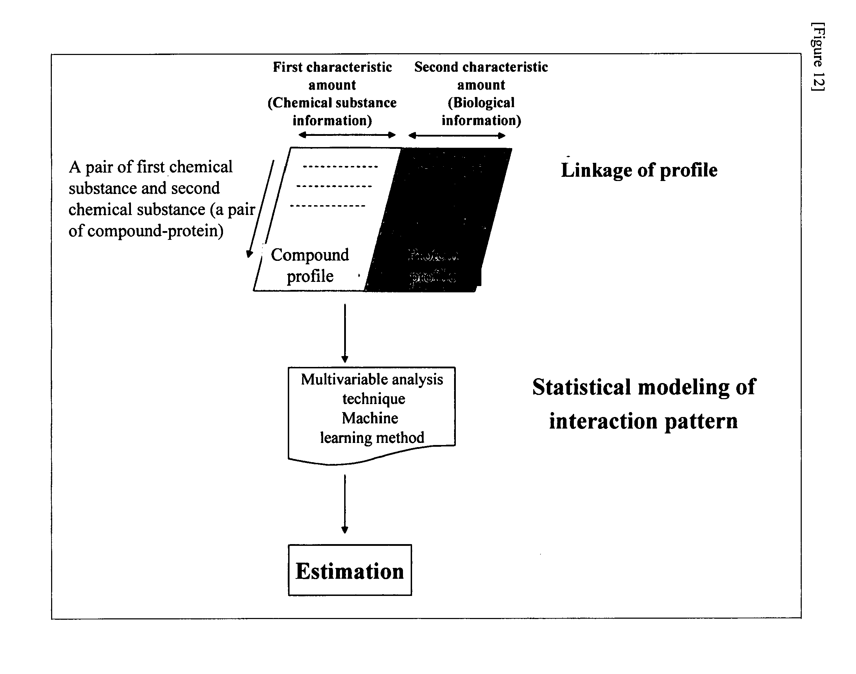

In addition, this invention in the specification can be used in the embodiment to show below it (figure 11 - figure 13 shows a conception diagram about the following explanation).

In another situation, this invention offers a data processing method to define an interaction pattern between the first chemical substance group and second chemical substance group as a statistical model, and this method defines the first space expressing coordinates in space of the first chemical substance group and the second space expressing coordinates in space of the second chemical substance group, and the first chemical substance group is characterized by the first characteristic amount, and the second chemical substance group is characterized by the second characteristic amount, (I) the first chemical substance group is a compound, and the second chemical substance group is a nucleic acid or protein or those complexes and the first said characteristic amount is expressed as a vector consisting of one or two more kinds of chemical compound information of the said first chemical substance, and the said second characteristc amount is expressed as vector consisting of one or two more kinds of chemical compound information of the said second chemical substance (As figure 13 says, the difinitions of chemical substances and charateristic amount are exchanged), the first said space and the second said space are data processing methods converted representation into by technique chosen from multivariate analysis technique, mechanic learning method and those equivalence of value methods group, and a correlation between the first above space and the second above space becomes greatest.

Here, not only linear conversions by CCA or PCA, but a non-linear conversion by the kernel function is included in map-transformation in this situation of this invention. Moreover it can be used in both (I) and (II), it can be used for searching for compounds which have activity on the specific protein, it offers knowledge about adverse effect of drug, too. And it can be used for creation of artificial protein (gene recombinant protein) which have effect on a specific compound.

It is a data processing method to predict interaction between the first chemical substance and the second chemical substance, and one enforcement form in this method includes following A) - D). A) A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1; B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction, C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion. D) A process of outputting the first chemical substance and the second chemical substance having more than the specific scores. Here , mapping is map-transforming by the multivariate analysis technique, the machine learning technique and those equivalence of value methods, using the function dereived map-transforming, in order to get the correlation between the first above space and the second above space becomes greatest. Furthermore, for example, 5% intentionality points to show intentionality point of statistics are raised, but, in D), the criterion about the specific score is not limited to these about "specific score" to appoint.

In addition, this data processing method offers a data processing method of producing a chemical substance having a desied characteristic amount or designing library, including A) - D), A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1; B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction, C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion. D) a process of choosing a desired characteristic amount in the second characteristic amount, E) A process of calculating a focused region in the map-transformed second space of the second chemical substance group having the desired characteristic amount, F) A process of calculateing a target region in the map-transformed first second space of the first chemical substance group having more than the specific scores with the second chemical substance in the said focused region, G) A process of choosing a first chemical substance or a first chemical substance group existing in the said target region.

In another situation, this invention is a data processing method to define a interaction pattern between the first chemical substance group and the second chemical substance group as a statistical model, comprising, the pair of the first chemical substance and the second chemical substance is expressed as the vector that is a combination of the first characteristic amount of the first chemical substance with the second characteristic amount of the second chemical substance, (I) the first chemical substance group is a compound, and the second chemical substance group is a nucleic acid or protein or those complexes, and the first characteristic amount is expressed as a vector consisting of the one kind chemical information of the said first chemical substance group, and the second characteristic amount is expressed as a vector consisting of the one kind biological information of the said second chemical substance group; or (II) the first chemical substance group is a nucleic acid or protein or those complexes, and the second chemical substance group is a compound, and the first characteristic amount is expressed as a vector consisting of the information of one kind of biological information of the said fist chemical substance group, and the second characteristic amount is expressed as a vector consisting of the one kind chemical information of the said second chemical substance group; the vector that is a combination of the first characteristic amount of the first chemical substance with the second characteristic amount of the second chemical substance, is map-transformed into a feature space by technique chosen from multivariate analysis technique, mechanic learning method and methods equivalent for those.

In one embodiment, this method is a data processing method to predict interaction between the first chemical substance and the second chemical substance in a data processing method of the description to above, including, A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according above; B) a process of mapping a vector that is a combination of the first characteristic amount of the first chemical substance to be estimated for interaction with the second characterictic amount of the second chemical substance to be estimated for interaction, into the feature space, by the said map-transforming, C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction. D) a process of outputting the first chemical substance and the second chemical substance having more than the specific scores. and the information of interaction is defined by the information chosen from existence / nothing of binding, biding activity, the pharmacology activity. Here, the "Interaction information" includes a dissociation constant Kd, concentration in 50 % inhibitory effect concentration IC50 and 50 % increase effect concentration EC50. In the case of drug development, Kd, IC50 and EC50 as the standard of the presence of binding, binding activity and pharmacological activity are micro mole order or nano mole order. Further, the score of probability for interaction is expressed with a distance from a boundary surface (hyperplane) for class classification (for example, a class of binding pair of compound and protein and a class of not-binding pair of compound and protein) within a feature space to the estimation target. The further the distance from the boundary surface becomes, the higher the probability for interaction becomes.

In another embodiment, this method is a data processing method to produce a chemical substance having a desied characteristic amount or to design library , including, A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4; B) a process of mapping a vector that is a combination of the first characteristic amount of the first chemical substance to be estimated for interaction with the second characterictic amount of the second chemical substance to be estimated for interaction, into the feature space, by the said map-transforming, C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction, D) a process of choosing a desired characteristic amount in the second characteristic amount, E) A process of calculating a focused region in the map-transformed second space of the second chemical substance group having the desired characteristic amount, F) A process of calculateing a target region in the map-transformed first second space of the first chemical substance group having more than the specific scores with the second chemical substance in the said focused region, G) A process of choosing a first chemical substance or a first chemical substance group existing in the said target region.

A data processor predicting interaction between the first chemical substance and the second chemical substance is offerd even if any data processing method of either situation of this invention which listed above is used. And, in another embodiment, a data processor to produce a chemical compound having desired characteristic amount or libraries by the above data processing methods is offered. These processors are enforced by the process of the present invention being carried out on an arithmetic unit provided for the processor.

In another embodiment, with a data processing method of this invention, a data processing program to produce a chemical compound having quantity of desired character or libraries is offered. In another embodiment, with one data handling of this invention method, a data processing program to predict interaction between the first chemical substance and the second chemical substance is offered. Such a method of implementation by letting a computer, a computer implement the recording medium that the computer reading that a program was stored is possible through tool (if, for example, it is CD - R, CD-R drive) reading a recording medium. The data processing program of this invention is carried out on a computer, a computer. In addition, in the specification, "a computer", "the computer" are used and show an electron machine performing information processing based on an order automatically. In addition, the computer keeps an input device, a storage device, an arithmetic unit, control equipment, an output device when it is representative. An input device is a device to use to forward data and information to a datamation system, and, for example, a keyboard, Mus musculus, tablet, a scanner are raised, but it is not limited to these. A storage device is a device memorizing the information which is necessary for a calculation, and memory, a disc, tape, CD-ROM / RAM, DVD are raised, but it is not limited to these. The arithmetic unit is moiety of a computer carrying out all arithmetic operations and Boolean operations, and CPU's are the examples of this. It is data buffer and an electronic device comprising the logical circuit, and there is the control equipment between a computer channel and input-output devices and, for example, controls the engineer which seems to be data transmission and tape rewind. An arithmetic unit and control equipment is called "a processor" in a mass, and there is it when it is used term with the arithmetic unit in the meaning of the processor again. The description of the program can give any language (for example, C + language, perl, Basic, html, XML, Pascal, FORTRAN) in the specificaion detailed statement in the field concerned, but it is not limited to them. In addition, it is understood that it points at a computer program at time called the program with the instant specification unless it is limited pariculary. In addition, it can be done storage (a memory) of the recording medium that computer reading is possible the data processing program of this invention. It is understood that it can be used in any form as far as it can be to record a program for a recording medium.

In addition, in another embodiment of this invention, this invention offers a chemosynthesis device which has a data processing program of this invention, and which synthesis the chemical substances or the chemical substances group predicted or produced by the data processing program or by the data processing machine of thi invention.

Furthermore, this invention offers a screening device which measures the second characteristic amount of the chemical substances in the first space, and which have a tool to choose chemical substances which have really desired activity, after synthesising the chemical substance group of the first space.

Furthermore, a desired chemical substance is composed by using the data processing program, the recording medium that computer can read, or the device of this invention.

A desired chemical substance is synthesised by using normal chemistry technology, based on knowledge obtainede by the data processing method of this invention.

The whole refers to the references such as scientific literature, a patent, the patent application quoted in an instant specification in an instant specification to a grade same as what was listed for each concreteness, and it is quoted.

The favorable embodiments of this invention were explained to be understood easily as things mentioned. In the following, this invention is explained based on example, but above-mentioned explanation and the following examples were offered to only the goal of the illustration, and did not offer it for the purpose of limiting this invention. Therefore, the range of this invention is limited only by claims without being limited to both enforcement form listed in an instant specification concretely and embodiment.

[Examples:]

Hereafter, the present invention will be explained with reference to the examples; however, the present invention shall not be limited to the examples mentioned below.

(Example 1: Screening in biological space and chemical space using CCA)

In order to evaluate the performance of the conventional method (PCA) and the present technique (CCA), in-silico screening was conducted using "integrated model of chemical space and biological space" constructed by both methods, respectively.

The figure shows a ROC curve, which is one of the methods to evaluate the estimation performance. This graph indicates that the more upper the curve is positioned, the better the estimation performance is, and since the curve of the present technique is positioned more upper than that of the conventional method, the estimation performance of the present technique is higher than that of the conventional method. The specific procedures are shown hereafter.

(Protocol)

1. Data of known proteins and compounds to be used for the formulation of integrated model were acquired from Drug Bank Database (http://redpoll.pharmacy.ualberta.ca/drugbank/), version released in August 2005.

2. For a mol file of all compound entries, 936 compound descriptors were calculated using Dragon X software. Herein, the number of calculated compounds is 3,079. In addition, for the purpose of CCA calculation, since the profile of the descriptor to be an attribute has to be independent, the descriptors having correlation with 0.8 or greater of coefficient of correlation were reduced to 300 descriptors with higher information volume.

3. For a fasta file of all protein entries, a 400-dimensional (amino acids: 20 types*20 types) profile composed of relative proportions of two successive amino acids by taking mismatch into consideration was calculated by the technique, which is similar to the technique to generate mismatch string kernel. Herein, the number of profiled proteins is 3,476. Further, the number of binding with the calculated compounds in above-mentioned 2. was 8,006.

4. For the evaluation of estimation performance, a 5-fold cross validation was used. In other words, 8,006 compound-protein binding data prepared in the above-mentioned 2. and 3. were randomly classified into 4:1, and CCA calculation and PCA calculation were conducted using 80 % of binding data as training data, and the coordinates of the biological space and the chemical space were formulated. The remaining 20 % of binding data was used as test data. Herein, a negative example of test data (not-binding data) generated a combination, which would not be bound with the combinations of compounds and proteins, which form binding data as a positive example, and the same number as the positive examples were randomly selected. The test data prepared as described above was mapped into the biological space and the chemical space formulated by the training data, respectively. For the mapping, a weighting factor matrix (in the case of PCA, principal component score coefficient matrix) is applied to a test data matrix by CCA or PCA calculation of the training data. For the proteins and compounds mapped to the biological space and the chemical space, the score was calculated, respectively.

5. When the test data is estimated as shown in the above-mentioned 4., a proportion for the one, which was able to be estimated to bind the binding data in actuality, is referred to as a true positive rate, and a proportion for the one, which happened to be estimated to bind the not-binding data in actuality, is referred to as a false positive rate. Herein, data having a specific estimation score (threshold) is regarded as positive, and data having a specific estimation score or less is regarded as negative. The threshold of the score was fluctuated based upon the compound-protein binding score estimated in CCA and PCA, and they are plotted as the false positive rate and the true positive rate (x,y) associated with the fluctuation and a ROC curve was created (Fig. 6).

(Example 2: Compound-GPCR interaction estimation technique)

A compound-GPCR interaction estimation technique was developed with the procedures mentioned below.

Furthermore, for references where data sets and analysis methods used in this example are described, reference numbers were marked in the text and a list of references was attached at the end of this example. These references are incorporated in the specification as references.

(#1 Collection of compound-protein interaction information)

Combinations of interacting compounds-GPCR, 5207 examples (compounds: 866, GPCR: 317) were collected from GLIDA (GPCR-LIgand DAtabase) [1], Drug Bank [2], IUPHAR Reeceptor database [3] and PDSP Ki database [4]. However, herein, human, mouse and rat GPCR were used, and GPCR was defined in accordance with GPCRDB [5]. Further, for the compounds, since structure information was required for the calculation of following descriptor, compounds registered in GLIDA and PubChem Compound [6] providing mol(sdf) format files were used.

(#2 Calculation of descriptor)

In order to express the compound and protein as a characteristic vector, each descriptor was calculated with the following methods:

- Chemical descriptor
  A descriptor regarding the structure and physical property of the compounds were calculated from the structures of collected compounds according to Dragonx ver. 1.2 [7]. In this study, a total of 929 descriptors in categories 1-10 (constitutional descriptors, topological descriptors, walk and path counts, connectivity indices, information indices, 2D autocorrelations, edge adjacency indices, Burden eigenvalue descriptors, topological charge indices and eigenvalue-based indices), categories 17-18 (functional group counts and atom-centered fragments) and category 20 (molecular properties), were calculated. Furthermore, descriptors depending upon the three-dimensional coordinates of molecular (categories 11-14), descriptors counting the number of functional groups and atom types (categories 15 and 16) and an electric charge descriptor (category 19) were not used herein. Subsequently, among these descriptors, ones where all compounds were calculated and outputted as the same value were removed, and 797 types of descriptors remained as a result were used hereafter.
- Protein descriptor
  This descriptor was calculated using a spectrum method [8] where mismatch was allowed. In this method, a protein sequence is broken down to an amino acid sequence with fixed length k, and calculation is conducted by counting the frequency of the amino acid sequence pattern with the length k where up to m mismatches are allowed appeared in this sequence. The inventors set (k,m) at (2,1). Therefore, the number of the descriptors to be calculated is 202 types of double amino acids where ond amino acid mismatch is allowed.

(#3 Structure of learning model by support vector machine (SVM))

SVM is a learning algorithm proposed by Vapnik et al. [9], and it is often used in many fields because of its high generalizing capability. SVM constructs a hyperplane so as to separate characteristic vectors of two different groups by a maximum margin. Herein, the maximum margin indicates the shortest distance from the separated hyperplane to each sample.

The inventors, for obtaining a hyperplane for separating whether or not the compound-protein interaction exists, a characteristic vector was constructed by combining the chemical descriptor and the protein descriptor corresponding to a positive example (interacting pattern) and a negative example (not interacting pattern), respectively, and constructed a learning model using SVM. However, for the negative example, because information of not-interacting pattern was not obtained, two descriptors were combined at random and the negative examples as many as the positive examples were generated. Herein, as the SVM library, codes of libsvm program [12] adopting "Sequential Minimal Optimization" algorithm [10, 11 ] were used. Once the SVM model is obtained, which class of interaction or no interaction a new vector (a pair of compound and protein) belongs to can be estimated. In addition, not only the determination, a method for sample scoring is also reported [13]. This is based upon a notion where a sample close to a separation plane would have a higher probability to be classified by accident compared to a sample far from the separation plane. The inventors scored and prioritized the possibility using this method in the interaction estimation between compound-protein.

(#4 Ligand estimation by compound structure similarity)

The inventors used the similarity of the compound calculated from the chemical descriptor as a ligand estimation method to be a model comparison. This similarity is a method for a general compound search, and it is said that this is a help to discover a lead compound [14]. In this study, 797 types of chemical descriptors calculated in #2 were analyzed regarding principal components, and compounds adjacent to the known ligand were sequentially scored on the principal component. The score of a pair of compound A and protein B is expressed with a similarity with the known ligand of the protein B the closest to the compound A. For the principle component, up to components with 80 % of accumulated contributing rate (30 principal components) were used. Further, as the similarity scale, a correlation coefficient (Pearson correlation coefficient) was used.

(#5 Evaluation of model by cross validation)

The estimation performance of a learning model was evaluated using n-fold cross-validation. In this validation, at first, all learning data sets are divided into n sub-sets with equal size. Subsequently, each sub-set is estimated using a classifier, which was learned and made with the remaining (n-1) sub-sets. Then, this operation is repeated so as to estimate all sub-sets only once and evaluation is conducted. As the scale of the estimation performance, the accuracy calculated with an expression mentioned below was used. The accuracy is expressed with the following expression.

$$Accuracy = (TP + TN)/(TP + TN + FP + FN)$$

Herein, TP, TN, FP and FN represent true positive, true negative, false positive and false negative, respectively.

Taking the score fluctuation by a negative example created with random combination into consideration, ten times of different data sets were created while a negative example was exchanged, and the 5-fold cross-validation was repeated and the model by the inventors was evaluated using a mean value of the accuracy. Subsequently, ROC analysis was conducted from the calculated interaction estimation score. Herein, in each evaluation, the ligand estimation method (#4) based upon the structure similarity of the compound was regarded as a comparison subject.

(#6 Ligand estimation of human β2 adrenaline receptor (β2AR)

The compound-GPCR interaction information collected by the inventors is only known as "strongly binding" according to the research up to now, and other majority of compound-GPCR interaction is unknown. The question by the inventors is whether or not a compound, which has been estimated as interaction, may not interact in the ligand search. Then, the inventors confirmed the relevancy between the interaction estimation score and the presence of interaction was confirmed according to in vitro binding inhibitory test.

Consequently, human β2AR was regarded as a target protein, and the ligand estimation was conducted using the prepared learning model. This receptor is a physiologically important gene for development of therapeutic drug as a target of asthma therapy. The subject compound for the ligand estimation was the above-mentioned 866 compounds known with the interaction with GPCR (however, a compound where the interaction with human β2AR was learned at the time of model structure was excluded). β2AR protein descriptors were combined with the chemical descriptors of these compounds, and data sets for estimation were obtained. Taking the score fluctuation due to the combination of negative examples into consideration, a trial of the learning and estimation was repeated 30 times while the negative examples were exchanged, and the maximum value of the obtained score was regarded as a final estimation score.

Next, the compounds in the top 50 of interaction estimation scores were further examined and tested. First, whether or not any report regarding the interaction with β2AR was checked according to references and patent search (SciFinder, PubMed).

Subsequently, among the compounds where no interaction information could be confirmed, acquirable compounds were verified using binding inhibitory test in vitro. In this test, a membrane fraction was prepared from the human β2AR forced expression cell strain, and a competitive inhibitory effect on [$^{125}$I]-Cyano pindolol, which is radioactive β2AR ligand, was confirmed.

Due to lack of information about no interaction, in the model by the inventors, randomly-generated pair of compound-protein is adopted as a negative example (no interaction) pattern. Consequently, it is necessary to confirm whether or not a compound with a low interaction estimation score does not really interact. Then, the inventors, for the bottom 50 of estimation scores, conducted the reference search and verification test similarly to the top 50 of estimation scores.

(Results)

(Evaluation of new ligand estimation model by cross validation)

First, the developed method and the conventional method were compared first. The characteristic vector of the compound-protein interaction pattern was regarded as an input of SVN classifier using the GPCR-ligand interaction information collected from public database, and a learning model was constructed. As a result of trying the 5-fold cross validation while exchanging a negative example 10 times, the estimation performance (accuracy) of the model developed by the inventors was 91.3 % +/- 0.3 %. As a comparison, for the conventional method based upon the compound similarity, the 5-fold cross validation was similarly conducted and the estimation performance 81.9 % +/- 0.3 %. Further, it became ascertained that the estimation performance of the model developed by the inventors is high according to the ROC curve, as well (Fig. 7A).

(Human β2AR ligand estimation)

Next, the new technique was applied to the new ligand estimation for human β2AR, and the validity was verified from the experiment. Further, whether or not a ligand, which is estimated only by the new technique, includes a compound having a new skeleton, which cannot be detected with the conventional method, was examined. The interaction estimation score with β2AR was calculated using the constructed model, for 866 types of GPCR ligands.

Among the top 50 of β2AR ligand candidates estimated by the new model, reports where 14 types of compounds that interact with β2AR were confirmed according to references and patent search (left (B-1) in Fig. 8). In addition, among the remaining compounds with unknown interaction, the in vitro binding inhibitory test was conducted for 21 acquirable types, and 17 types of compounds showed the interaction ($10^{-5}$ M < $IC_{50}$ < $10^{-3}$ M) (right (B-1) in Fig. 8). The rate of hit in the test was up to 81 % (17/21), and a high estimation hitting ratio is shown herein.

In the meantime, for the bottom 50 of compounds, compounds reported as β2AR ligand were not confirmed in references and patent search (left (B-2) in Fig 7B). In addition, for 9 acquirable compounds among the remainder compounds, the in vitro binding test was conducted, and two compounds showed the same level of strength; however, the remaining 7 compounds did not show any interaction (right (B-1) in Fig 7B).

Fig 7C is a graph where these estimation results are compared to that with the conventional method. Nearly one-half of compounds where the interaction was confirmed with the tests shows lower scores with the conventional method based upon the structure similarity of compounds. In actuality, these compounds have various skeletons (left of Fig 7C), which are different from those in the structure of the typical β2AR agonist (catecholamine skeleton, isoprenaline derivative) and the structure of the β2AR antagonist (arylalkyl amine derivative), and this can be considered as a ligand group, which cannot be discovered with the conventional method based upon the structure similarity of compounds. In other words, it appears that the new model based upon the interaction information can accurately estimate the relationship where a compound with various structures acts on the same protein. Further, compounds, conventionally known as compounds acting on a peptide receptor, such as neuropeptide receptor antagonist, were included in these compounds; however, it was confirmed that they also interact with distantly-related β2AR according to the test.

[Table 1]

[References]

[1] Okuno Y, Yang J, Taneishi K, Yabuuchi H & Tsujimoto G. GLIDA: GPCR -ligand database for chemicalgenomic drug discovery. Nucleic Acids Res. 34,D673 - 7(2006).

[2] Fredholm B.B., Fleming W.W., Vanhoutte P.M. & Godfraind T. The role of pharmacolo gy in drug discovery.Nat. Rev. Drug Discov. 1,237 -8(2002).

[3] Wishart D.S., Knox C, Guo A.C., Shrivastava S. Hassanali M, Stothard P, Chang Z & Woolsey J.DrugBank: a comprehensive resource for in silico drug discovery and exploration.NucleirAcids Res. 34, D668 - 72 (2006).

[4] Roth B.L., Kroeze W.K., Patel S & Lopez E. The Multiplicity of Serotonin Receptors: Uselessly diversemolecules or an embarrassment of riches? The Neuroscientist 6,252 -262 (2000).

[5] Horn F, Bettler E, Oliveira L, Camp agne F. Cohen F.E. & Vriend G. GPCRDB information system for G protein -coupled receptors. Nucleic Acids Res. 31, 294 - 7(2003).

[6] Wheeler D.L., Barrett T. Benson D.A., Bryant S.H., Canese K. Chetvernin V, Church D.M., et. al.Database resources of the National Center for Biotechnology Information.Nucleic Acids Res. 34, D173 - 80(2006).

[7] DRAGON software is available at http://www.talete.mi.it/main_net.htm

[8] Leslie C.S., Eskin E, Cohen A. Weston J & Noble WS. Mismatch string kernels for discriminativeprotein classi-fication. Bioinformatics 20,467 - 76(2004).

[9] Vapnik V.N The Nature of Statistical Learning Theory Springer: New York, (1995)

[10] Platt J. Fast Training of Support Vector Machines using Sequential Minimal Optimization;Microsoft Research Technical Report MSRTR - 98 - 14 (1998).

[11] Keerthi S.S., Shevade S.K., Bhattacharyya C, & Murthy K.R.K. Improvements to Platt's SMO Algorithm for SVM Classifier Design. Neural Comput. 13,637 - 649(2001).

[12] Chang C.C. & Lin C.J. L IBSVM: a library for support vector machines.Software is available at http://www.csie.ntu.oou.tw/~cjlin/libsvm

[13] Platt J. Probabilistic outputs for support vector machines and comparison to regularized likelihood methods.Advances in Large Margin Classifiers (pp.61 - 74).(1999)

[14] OpreaT.I. Chemical space navigation in lead discovery. Curr. Opin. Clem .Biol. 6,384-9 (2002)

[An advantage possibility in the industry]

By this technology, I largely lower the cost of the new medicine development in the field of the drug development in particular and can shorten it again for the research and development cycle.
The market can hereby send better medicine off conventionally in a short period.
In addition, I can expect contribution called the reduction of the medical cost burden socially by lowering the ratio of research and development expenses to occupy to medicine manufacture cost.

**Claims**

1. A data processing method to define a interaction pattern between the first chemical substance group and the second chemical substance group as a statistical model,

the first space expressing coordinates of the first chemical substance group and the second space expressing coordinates of the second chemical substance group are defined, and the first chemical substance group is **characterized by** the first characteristic amount, and the second chemical substance group is **characterized by** the second characteristic amount,

(I) the first chemical substance group is a compound, and the second chemical substance group is a nucleic acid or protein or those complexes, and the first characteristic amount is expressed as a vector consisting of the one kind chemical information of the said first chemical substance group, and the second characteristic amount is expressed as a vector consisting of the one kind biological information of the said second chemical substance group; or

(II) the first chemical substance group is a nucleic acid or protein or those complexes, and the second chemical substance group is a compound, and the first characteristic amount is expressed as a vector consisting of the information of one kind of biological information of the said fist chemical substance group, and the second characteristic amount is expressed as a vector consisting of the one kind chemical information of the said second chemical substance group;

the said first space and the said second space are map-transformed by technique chosen from multivariate analysis technique, mechanic learning method and methods equivalent for those,

the coordinates in the said first space and the coordinates in the said second space are map-transformed by technique chosen from multivariate analysis technique, mechanic learning method and methods equivalent for those, and the map-transformed first space coordinates and the map-transformed second space coordinates are difined.

2. A data processing method to predict interaction between the first chemical substance and the second chemical substance in a data processing method of the description to claim 1, including,

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1;

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) A process of outputting the first chemical substance and the second chemical substance having more than the specific scores.

3. A data processing method to produce a chemical substance having a desied characteristic amount or to design library in a data processing method of the description to claim 1, including,

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1;

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.
D) a process of choosing a desired characteristic amount in the second characteristic amount,
E) A process of calculating a focused region in the map-transformed second space of the second chemical substance group having the desired characteristic amount,
F) A process of calculateing a target region in the map-transformed first second space of the first chemical substance group having more than the specific scores with the second chemical substance in the said focused region,
G) A process of choosing a first chemical substance or a first chemical substance group existing in the said target region.

4. A data processing method to define a interaction pattern between the first chemical substance group and the second chemical substance group as a statistical model, comprising,
the pair of the first chemical substance and the second chemical substance is expressed as the vector that is a combination of the first characteristic amount of the first chemical substance with the second characteristic amount of the second chemical substance,

(I) the first chemical substance group is a compound, and the second chemical substance group is a nucleic acid or protein or those complexes, and the first characteristic amount is expressed as a vector consisting of the one kind chemical information of the said first chemical substance group, and the second characteristic amount is expressed as a vector consisting of the one kind biological information of the said second chemical substance group; or
(II) the first chemical substance group is a nucleic acid or protein or those complexes, and the second chemical substance group is a compound, and the first characteristic amount is expressed as a vector consisting of the information of one kind of biological information of the said fist chemical substance group, and the second characteristic amount is expressed as a vector consisting of the one kind chemical information of the said second chemical substance group;

the vector that is a combination of the first characteristic amount of the first chemical substance with the second characteristic amount of the second chemical substance, is map-transformed into a feature space by technique chosen from multivariate analysis technique, mechanic learning method and methods equivalent for those.

5. A data processing method to predict interaction between the first chemical substance and the second chemical substance in a data processing method of the description to claim 4, including,

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4;
B) a process of mapping a vector that is a combination of the first characteristic amount of the first chemical substance to be estimated for interaction with the second characterictic amount of the second chemical substance to be estimated for interaction, into the feature space, by the said map-transforming,
C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction.
D) a process of outputting the first chemical substance and the second chemical substance having more than the specific scores. and the information of interaction is defined by the information chosen from existence / nothing of binding, biding activity, the pharmacology activity.

6. A data processing method to produce a chemical substance having a desied characteristic amount or to design library in a data processing method of the description to claim 4, including,

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4;
B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction,

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

7. A data processing device to predict interaction between the first chemical substance and the second chemical substance, using a data processing method of the description to claim 1, has an arithmetic device and make the said arithmetic device run the method including A)-D);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1;

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) A process of outputting the first chemical substance and the second chemical substance having more than the specific scores.

8. A data processing device to produce a chemical substance having a desied characteristic amount or to design library, using a data processing method of the description to claim 1, has an arithmetic device and make the said arithmetic device run the method including A)-G);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1;

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

9. A data processing device to predict interaction between the first chemical substance and the second chemical substance, using a data processing method of the description to claim 4, has an arithmetic device and make the said arithmetic device run the method including A)-D);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4;
B) a process of mapping a vector that is a combination of the first characteristic amount of the first chemical substance to be estimated for interaction with the second characterictic amount of the second chemical substance to be estimated for interaction, into the feature space, by the said map-transforming,
C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction.
D) a process of outputting the first chemical substance and the second chemical substance having more than the specific scores.

and the information of interaction is defined by the information chosen from existence / nothing of binding, biding activity, the pharmacology activity.

10. A data processing device to produce a chemical substance having a desied characteristic amount or to design library, using a data processing method of the description to claim 4, has an arithmetic device and make the said arithmetic device run the method including A)-G);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4;
B) a process of mapping a vector that is a combination of the first characteristic amount of the first chemical substance to be estimated for interaction with the second characterictic amount of the second chemical substance to be estimated for interaction, into the feature space, by the said map-transforming,
C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction,
D) a process of choosing a desired characteristic amount in the second characteristic amount,
E) A process of calculating a focused region in the map-transformed second space of the second chemical substance group having the desired characteristic amount,
F) A process of calculateing a target region in the map-transformed first second space of the first chemical substance group having more than the specific scores with the second chemical substance in the said focused region,
G) A process of choosing a first chemical substance or a first chemical substance group existing in the said target region.

11. A data processing program to predict interaction between the first chemical substance and the second chemical substance, using a data processing method of the description to claim 1, make calculator run the method including A)-D);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1;
B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,
C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) A process of outputting the first chemical substance and the second chemical substance having more than the specific scores.

12. A data processing program to predict interaction between the first chemical substance and the second chemical substance, using a data processing method of the description to claim 1, make calculator run the method including A)-G);

A) Regarding the first chemical substance group and the second chemical substance group, which have been known as interacting with each other, a process of map-transforming the first space expressing coordinates of the first chemical substance group, and the second space expressing coordinates of the second chemical substance group, using the data processing method according to claim 1;

B) a process of mapping the first chemical substance into the first space by map-transforming a vector consisting of the first characteristic amount of the first chemical substance to be estimated for interatcion, and of mapping the second chemical substance into the second space by map-transforming a vector consisting of the second characteristic amount of the second chemical substance to be estimated for interaction,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interatcion.

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

13. A data processing program to predict interaction between the first chemical substance and the second chemical substance, using a data processing method of the description to claim 4, make calculator run the method including A)-D);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4;

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction.

D) a process of outputting the first chemical substance and the second chemical substance having more than the specific scores.

and the information of interaction is defined by the information chosen from existence / nothing of binding, biding activity, the pharmacology activity.

14. A data processing program to predict interaction between the first chemical substance and the second chemical substance, using a data processing method of the description to claim 4, make calculator run the method including A)-G);

A) regarding a pair of the first chemical substance group and the second chemical substance group, which have information of a desired interaction, a process of map-transforming a vector that is a combination of the first characteristic amount of the first chemical substance with the second characteristic amount of the second chemical substance, into the feature space, using the data processing method according to claim 4;

B) a process of mapping a vector that is a combination of the first characteristic amount of the first chemical substance to be estimated for interaction with the second characterictic amount of the second chemical substance to be estimated for interaction, into the feature space, by the said map-transforming,

C) a process of calculating a score of probability which the first chemical substance and the second chemical substance interact each other according to the map-transformed coordinate position of the first chemical substance to be estimated for interaction and the map-transformed coordinate position of the second chemical substance to be estimated for interaction,

D) a process of choosing a desired characteristic amount in the second characteristic amount,

E) A process of calculating a focused region in the map-transformed second space of the second chemical substance group having the desired characteristic amount,

F) A process of calculateing a target region in the map-transformed first second space of the first chemical substance group having more than the specific scores with the second chemical substance in the said focused region,

G) A process of choosing a first chemical substance or a first chemical substance group existing in the said target region.

15. A recording medium that stored the data processing program according to any one of claims 11-14, that computer can read.

16. The chemosynthesis device

that has the data processing program according to any one of claim 11-14, and synthesize a chemical substance or a chemical substance group which is prediceted or produced by the said data processing program; or

that synthesiza a chemical substance or a chemical substance group which is prediceted or produced by the data processing device according to any one of claim 7-10

17. A screeing device with the data processing program according to any one of claim 11-14 that has a way to synthesize a chemical substance in the frist space, assay the second cheracteristic amount of the chemical substance in the said first space, and actually choose the chemical substance which have desired activity.

18. A chemical substance which is produced by the method data processing program according to any one of calim 11-14, a recording medium of claim 15, or the device of claim 16 or 17.

[Figure 1]

Fig.1

[Figure 2]

EP 2 031 528 A1

# In silico screening

*Query compound*
*(chemical structure)*

Chemical space

Biological space

*Estimation of*
*target gene*

**Fig.2**

[Figure 3]

EP 2 031 528 A1

# In silico screening

_Lead compound estimation_

Chemical space

Biological space

_Query gene (protein)_
_(sequence structure)_

**Fig.3**

[Figure 4]

EP 2 031 528 A1

**Area corresponding to biological area
[Biologically Relevant Chemical Space]**
(Correlation between chemical space and biological space)

Compound library design

Chemical space

Limited biological space

Fig.4

FIG 5

## Construction method for integrated model of chemical space and biological space

Conventional method (principal component analysis (PCA))

Y

...2

PCA I

X

Space is defined so as to maximize dispersion only in chemical space

Technique of present invention (canonical correlation analysis (CCA))

Space is defined so as to maximize correlation between chemical space nd biological space

protB    protC
protA

protD    protE

* Chemical space with higher correlation with biological space is useful.

[Figure 6]

Fig.6

[Figure 7A]

Fig.7A

[Figure 7B]

Fig.7B

[Figure 7C]

Fig. 7C

[Figure 8]

EP 2 031 528 A1

# Chemical library design

PubChem
6,391,005

Library design

Biologically relevant
chemical library

**DrugBank
Chemcals
3079**

Chemical
space

**DrugBank
Proteins
3476**

Proteome space

Fig.8

[Figure 9]

## Library example: Bioactivity annotation of PubChem compounds  G.O. Function

| | score 27 | score 26 | score 25 | score 24 | score 23 | score 22 | score 21 |
|---|---|---|---|---|---|---|---|
| motor activity | 0 | 0 | 0 | 12859 | 58294 | 146521 | 30441 |
| catalyticactivity | 0 | 132 | 7498 | 91872 | 808264 | 2220698 | 806438 |
| helicaseactivity | 0 | 0 | 0 | 12763 | 67267 | 197040 | 44202 |
| signaltransduceractivity | 198 | 94476 | 633290 | 1512369 | 2068061 | 2517613 | 474165 |
| receptoractivity | 198 | 99111 | 677597 | 1623305 | 2387872 | 3033699 | 726701 |
| structural molecule activity | 0 | 119 | 3285 | 24141 | 233610 | 587076 | 147335 |
| transporteractivity | 0 | 17186 | 424389 | 1075678 | 1577678 | 2119350 | 468872 |
| carrieractivity | 0 | 12916 | 325688 | 804791 | 1032175 | 1335641 | 223948 |
| binding | 77 | 52722 | 490419 | 1599411 | 3075508 | 3886455 | 2047957 |
| electrontransporteractivity | 0 | 128 | 310 | 18706 | 348653 | 904792 | 220589 |
| proteinbinding | 0 | 28167 | 505062 | 1217307 | 1779455 | 2617458 | 752057 |
| molecular_functionunknown | 0 | 0 | 1 | 25707 | 74223 | 275611 | 49685 |
| aromataseactivity | 0 | 0 | 0 | 123 | 7033 | 39455 | 6987 |
| protein transporteractivity | 0 | 0 | 0 | 333 | 79719 | 77269 | 9330 |
| integraseactivity | 0 | 119 | 2565 | 11430 | 45211 | 193815 | 42182 |
| iontransporteractivity | 0 | 15869 | 330974 | 778901 | 957776 | 1167142 | 180847 |
| channel or pore class transporter acti | 0 | 813 | 24053 | 500237 | 1376542 | 2203510 | 393771 |
| antioxidantactivity | 0 | 0 | 0 | 6788 | 21683 | 96815 | 13285 |
| oxidoreductaseactivity | 0 | 2913 | 14793 | 324305 | 1419636 | 2695267 | 909669 |
| transferaseactivity | 0 | 672 | 10776 | 149433 | 951290 | 2245088 | 979300 |
| hydrolaseactivity | 0 | 12419 | 98644 | 359396 | 1276409 | 2848843 | 1203669 |
| lyaseactivity | 77 | 10685 | 17390 | 51148 | 568656 | 1351083 | 379692 |
| isomeraseactivity | 0 | 0 | 193 | 15063 | 234557 | 843863 | 219701 |
| ligaseactivity | 0 | 0 | 6 | 19484 | 315544 | 1100592 | 330559 |
| enzymeregulatoractivity | 0 | 1114 | 25007 | 55681 | 262072 | 576435 | 116670 |
| transcriptionregulatoractivity | 0 | 3822 | 36823 | 92885 | 248734 | 857861 | 159525 |
| translationregulatoractivity | 0 | 0 | 19 | 29088 | 44889 | 65483 | 13644 |

Fig.9

EP 2 031 528 A1

[Figure 10]

EP 2 031 528 A1

**Library example: Bioactivity annotation of PubChem compounds  G.O. Process**

| | score 27 | score 26 | score 25 | score 24 | score 23 | score 22 | score 21 |
|---|---|---|---|---|---|---|---|
| biological_process unknown | 0 | 15996 | 40887 | 15286 | 16059 | 59686 | 6338 |
| development | 0 | 28342 | 253537 | 716385 | 1308320 | 1943767 | 364059 |
| physiological process | 0 | 1403 | 195556 | 916314 | 1875148 | 2873385 | 569565 |
| behavior | 198 | 34605 | 355924 | 723085 | 639465 | 741478 | 103319 |
| metabolism | 0 | 540 | 2211 | 214126 | 1235709 | 2301389 | 665608 |
| catabolism | 0 | 2180 | 10054 | 11156 | 105197 | 316669 | 68674 |
| biosynthesis | 0 | 0 | 49 | 15710 | 315536 | 1014521 | 300801 |
| pathogenesis | 0 | 2180 | 11384 | 67318 | 176416 | 588308 | 132143 |
| cellular process | 198 | 97030 | 741665 | 1731806 | 2718587 | 3446377 | 1077203 |
| cell differentiation | 0 | 12 | 1595 | 122701 | 788274 | 1410128 | 215008 |
| macromolecule metabolism | 0 | 78 | 4444 | 66108 | 372248 | 1116652 | 439101 |
| secretion | 0 | 1 | 11480 | 70182 | 56843 | 35007 | 4822 |
| regulation of biological process | 0 | 11402 | 189000 | 711117 | 1315999 | 1676585 | 267610 |
| cellular physiological process | 77 | 61946 | 775127 | 1953107 | 3400815 | 3959376 | 2351247 |
| response to stimulus | 0 | 2311 | 211647 | 986589 | 2113675 | 2966214 | 903314 |

Fig.10

[Figure 11]

[Figure 12]

EP 2 031 528 A1

First characteristic
amount
(Chemical substance
information)

Second characteristic
amount
(Biological
information)

A pair of first chemical
substance and second
chemical substance (a pair
of compound-protein)

Compound
profile

Linkage of profile

Multivariable analysis
technique
Machine
learning method

Statistical modeling of
interaction pattern

Estimation

[Figure 13]

**First space : Compound group**

**Second space : Protein group**

**First space : Protein group**

**Second space : Compound group**

**INTERNATIONAL SEARCH REPORT**

| International application No. |
| --- |
| PCT/JP2007/060736 |

A. CLASSIFICATION OF SUBJECT MATTER
*G06F19/00*(2006.01)i, *C12N15/00*(2006.01)i

According to International Patent Classification (IPC) or to both national classification and IPC

B. FIELDS SEARCHED

Minimum documentation searched (classification system followed by classification symbols)
G06F19/00, C12N15/00

Documentation searched other than minimum documentation to the extent that such documents are included in the fields searched
Jitsuyo Shinan Koho        1922-1996    Jitsuyo Shinan Toroku Koho    1996-2007
Kokai Jitsuyo Shinan Koho  1971-2007    Toroku Jitsuyo Shinan Koho    1994-2007

Electronic data base consulted during the international search (name of data base and, where practicable, search terms used)
JSTPlus(JDream2), JMEDPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| A | JP 2002-530727 A (Glaxo Group Ltd.),<br>17 September, 2002 (17.09.02),<br>Full text; all drawings<br>& US 2002/0077754 A1    & US 2002/0052694 A1<br>& EP 1153358 A          & WO 2000/025106 A2<br>& AU 1331700 A          & CA 2346235 A | 1-17 |
| A | WO 2001/069440 A1 (Chugai Pharmaceutical Co., Ltd.),<br>20 September, 2001 (20.09.01),<br>Full text; all drawings<br>& US 2002/0049771 A1    & EP 1283479 A1<br>& AU 4108101 A | 1-17 |
| A | Kazuyuki SHIMIZU, "Seimei System Kaiseki no Tameno Sugaku", Corona Publishing Co., Ltd.,<br>30 June, 1999 (30.06.99), pages 117 to 130 | 1-17 |

☐ Further documents are listed in the continuation of Box C.          ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>16 August, 2007 (16.08.07) | Date of mailing of the international search report<br>28 August, 2007 (28.08.07) |
| --- | --- |
| Name and mailing address of the ISA/<br>Japanese Patent Office | Authorized officer |
| Facsimile No. | Telephone No. |

Form PCT/ISA/210 (second sheet) (April 2005)

## INTERNATIONAL SEARCH REPORT

| International application No. |
|---|
| PCT/JP2007/060736 |

**Box No. II   Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: 18
   because they relate to parts of the international application that do not comply with the prescribed requirements to such an extent that no meaningful international search can be carried out, specifically:
   It is unclear what specific compounds are involved in the scope of "chemicals" as defined in claim 18 and what compounds are not.  Such being the case, no meaningful international search can be carried out on this claim.

3. ☐ Claims Nos.:
   because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the
☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002530727 PCT

**Non-patent literature cited in the description**

- *Nikkei industry newspaper,* 29 November 2004
- **YOSHIFUMI FUKUNISHI ; YOSHIAKI MIKAMI ; HARUKI NAKAMURA.** Thefillingpotential method:A method for estimating the free energy surfaceforprotein-liganddocking. *J.Phys. Chem. B.,* 2003, vol. 13210 (107), 13201
- **SHOICHET, B.K. ; D.L. BODIAN ; I.D. KUNTZ.** Molecular docking usingshapedescriptors. *J.Comp. 1992. Chem.,* 1992, vol. 13 (3), 380-397
- **JONES G ; WILLETTP ; GLEN RC ; LEACH AR ; TAYLOR R.** Development and validation ofa geneticalgorithm for flexible docking. *J Mol Biol.,* 1997, vol. 267 (3), 727-748
- **RAREY M ; KRAMERB ; LENGAUER T.** Time-efficient docking of flexibleligands intoactive sites ofproteins. *Proc Int Conf IntellSyst Mol Biol.,* 1995, vol. 3, 300-308
- **OKUNO, Y. ; YANG,J. ; TANEISHI, K. ; YABUUCHI,H. ; TSUJIMOTO, G.** GLIDA:GPCR-Liganddatabasefor Chemical Genomic Drug Discovery. *Nucleic AcidsResearch,* 2006, vol. 34, D673-677
- Chemical Space. *Nature,* vol. 432 (7019), 823-865
- *Current Opinion in Chemical Biology,* 2005, vol. 9, 296-303
- Handbook of Chemoinformatics: From Data to Knowledge Gasteiger. John Wiley, October 2003
- **M.HASSAN et al.** *Molecular Diversity,* 1996, vol. 2, 64
- **M.J. MCGREGOR et al.** *J.Chem. Inf. Comput. Sci.,* 1999, vol. 39, 569
- **R.D. BROWN.** *Perspectives in Drug Discovery and Design,* 1997, vol. 7, 8, 31
- **R.D. BROWN et al.** *J. Chem. Inf. Comput. Sci.,* 1996, vol. 36, 572
- **R.D. BROWN et al.** *J. Chem. Inf. Comput. Sci.,* 1996, vol. 37, 1
- **D.E. PATTERSON et al.** *J. Med. Chem.,* 1996, vol. 39, 3049
- **S.K. KEARSLEY et al.** *J. Chem. Inf. Comput. Sci.,* 1996, vol. 36, 118
- **M.J. MCGREGOR et al.** *J. Chem. Inf. Comput. Sci.,* 1997, vol. 37, 443
- A High - Resolusion Multimode Digital Microscope System. METHODS IN CELL BIOLOGY. 1998, vol. 56, 185-215
- **INOUE ; SPRING.** Video Miroscopy. 1997
- **KAZUYUKI SHIMIZU.** Mathematics for Life System Analysis. Corona Publishing Co., Ltd, 1999
- **P. W. SPRAGUE et al.** Perspectives in Drug Discovery and Design. ESCOM Science Publishers B. V, 1995, vol. 3, 1
- **D. BARNUM et al.** *J. Chem. Inf. Comput. Sci.,* 1996, vol. 36, 563
- **J. GREENE et al.** *J. Chem. Inf. Comput. Sci.,* 1994, vol. 34, 1297
- **P. W. SPRAGUE et al.** Perspectives inDrug Discovery and Design. ESCOM Science Publishers B. V, 1995, vol. 3, 1
- **J. GREENE et al.** J. Chem. Inf. Comput. Sci., ChemDiverse module. ChemicalDesign Ltd, 1994, vol. 34, 1, 297
- **S. D. PICKETT et al.** *J. Chem. Inf. Comput. Sci.,* 1996, vol. 36, 1214
- **A.C. GOOD et al.** *J. Comput. Aided Mo. Des.,* 1995, vol. 9, 373
- **J. S. MASON et al.** *Perspective in Drug Discovery and Design,* 1997, vol. 7, 8, 85
- **S. D. PICKETT et al.** *J. Chem. Inf. Comput. Sci.,* 1998, vol. 38, 144
- **C. M. MURRAY et al.** *J. Chem. Inf. Comput. Sci.,* 1999, vol. 39, 46
- **J. S. MASON et al.** *J. Med. Chem.,* 1999, vol. 39, 46
- **R. NILAKANTAN et al.** *J. Chem. Inf. Comput. Sci.,* 1993, vol. 33, 79
- **X. CHEN et al.** *J. Chem. Inf. Comput. Sci.,* 1998, vol. 38, 1054
- **ALBERTS.** Molecular Biology of the Cell. 1994
- **CANTOR ; SCHIMMEL.** *Biophysical Chemistry Part I : refering to The Conformation of Biological Macromolecules,* 1980

- **P. J. GOODFORD.** A Computational Procedure for Determining Energetically Favorable Binding Sites on Biologically Important Macromolecules. *J. Med. Chem.,* 1985, vol. 28, 849-857
- **A. MIRANKER.** Functionality Maps of Binding Sites. *A Multiple Copy Simultaneous Search Method Proteins : Structure, Function and Genetics,* 1991, vol. 11, 29-34
- **D. S. GOODSELL.** Automated Docking of Substrates to Proteins by Simulated Annealing. *Proteins : Structure, Function, and Genetics,* 1990, vol. 8, 195-202
- **I. D. KUNTZ.** A Geometric Approach''to Macromolecule - Ligand Interactions. *J. Mol. Biol.,* 1982, vol. 161, 269-288
- **E.R. FELDER.** *Chimia,* 1994, vol. 48, 512-541
- **GALLOP et al.** *J. Med. Chem.,* 1994, vol. 37, 1233-1251
- **R.A. HOUGHTEN.** *Trends Genet.,* 1993, vol. 9, 235-239
- **HOUGHTEN et al.** *Nature,* 1991, vol. 354, 84-86
- **LAM et al.** *Nature,* 1991, vol. 354, 82-84
- **CARELL et al.** *Chem. Biol.,* 1995, vol. 3, 171-183
- **MADDEN et al.** *Perspectives in Drug Discovery and Design,* 1995, vol. 2, 269-282
- **CWIRLA et al.** *Biochemistry,* 1990, vol. 87, 6378-6382
- **BRENNER et al.** *Proc. Natl. Acad. Sci. USA,* 1992, vol. 89, 5381-5383
- **GORDON et al.** *J. Med. Chem.,* 1994, vol. 37, 1385-1401
- **LEBL et al.** *Biopolymers,* 1995, vol. 37, 177-198
- **D.K. AGRAFIOTIS et al.** *Molecular Divesity,* 1999, vol. 4, 1
- **U. EICHLER et al.** *Drugs of the Future,* 1999, vol. 24, 177
- **A. K. GHOSE et al.** *J. Comb. Chem.,* 1999, vol. 1, 55
- **E. J. MARTIN et al.** *J. Comb. Chem.,* 1999, vol. 1, 32
- **P. R. MENARD et al.** *J. Chem. Inf. Comput. Sci.,* 1998, vol. 38, 1204
- **R. A. LEWIS et al.** *J. Chem. Inf. Comput. Sci.,* 1997, vol. 37, 599
- **M. HASSAN et al.** *Molecular Divesity,* 1996, vol. 2, 64
- **M. J. MCGREGOR et al.** *J. Chem. Inf. Comput. Sci.,* 1999, vol. 39, 569
- **R. D. BROWN.** *Perspectives in Drug Discovery and Design,* 1997, vol. 7, 8, 31
- **T. W. ANDERSON.** An Introduction to Multivariate Statistical Analysis. Wiley & Sons, 1984
- **H. HOTELLING.** Relations between two sets of variates. *Biometrika,* 1936, vol. 28, 321-377
- **S. AKAHO.** A kernel method for canonical correlation analysis. *International Meeting of Phychometric Society,* 2001
- A training algorithm for optimal margin classifiers. **B. E. BOSER ; I. M. GUYON ; V. N. VAPNIK.** 5th Annual ACM Workshop on COLT. 144-152
- **OKUNO Y ; YANG J ; TANEISHI K ; YABUUCHI H ; TSUJIMOTO G.** GLIDA: GPCR -ligand database for chemical genomic drug discovery. *Nucleic Acids Res.,* 2006, vol. 34, 673-7
- **FREDHOLM B.B. ; FLEMING W.W. ; VANHOUTTE P.M. ; GODFRAIND T.** The role of pharmacolo gy in drug discovery. *Nat. Rev. Drug Discov.,* 2002, vol. 1, 237-8
- **WISHART D.S. ; KNOX C ; GUO A.C. ; SHRIVASTAVA S. ; HASSANALI M ; STOTHARD P ; CHANG Z ; WOOLSEY J.** DrugBank: a comprehensive resource for in silico drug discovery and exploration. *NucleirAcids Res.,* 2006, vol. 34, 668-72
- **ROTH B.L. ; KROEZE W.K. ; PATEL S ; LOPEZ E.** The Multiplicity of Serotonin Receptors: Uselessly diverse molecules or an embarrassment of riches?. *The Neuroscientist,* 2000, vol. 6, 252-262
- **HORN F ; BETTLER E ; OLIVEIRA L ; CAMP AGNE F. ; COHEN F.E. ; VRIEND G.** GPCRDB information system for G protein -coupled receptors. *Nucleic Acids Res.,* 2003, vol. 31, 294-7
- **WHEELER D.L. ; BARRETT T. ; BENSON D.A. ; BRYANT S.H. ; CANESE K. ; CHETVERNIN V ; CHURCH D.M.** Database resources of the National Center for Biotechnology Information. *Nucleic Acids Res.,* 2006, vol. 34, 173-80
- **LESLIE C.S. ; ESKIN E ; COHEN A. ; WESTON J ; NOBLE WS.** Mismatch string kernels for discriminative protein classification. *Bioinformatics,* 2004, vol. 20, 467-76
- **VAPNIK V.N.** The Nature of Statistical Learning Theory. Springer, 1995
- **PLATT J.** Fast Training of Support Vector Machines using Sequential Minimal Optimization. *Microsoft Research Technical Report MSRTR - 98 - 14,* 1998
- **KEERTHI S.S. ; SHEVADE S.K. ; BHATTACHARYYA C ; MURTHY K.R.K.** Improvements to Platt's SMO Algorithm for SVM Classifier Design. *Neural Comput.,* 2001, vol. 13, 637-649
- **CHANG C.C. ; LIN C.J.** *L IBSVM: a library for support vector machines,* http://www.csie.ntu.oou.tw/~cjlin/libsvm
- **PLATT J.** Probabilistic outputs for support vector machines and comparison to regularized likelihood methods. *Advances in Large Margin Classifiers,* 1999, 61-74
- **OPREAT.I.** Chemical space navigation in lead discovery. *Curr. Opin. Clem .Biol.,* 2002, vol. 6, 384-9